# FASCICULE DE BREVET EUROPEEN

(11) **EP 1 651 257 B1**
(45) Date de publication et mention de la délivrance du brevet: **17.02.2016**
(21) Numéro de dépôt: 04763487.8
(22) Date de dépôt: 25.07.2004
(51) Int. Cl.: A61K 39/00, A61K 39/385, A61P 35/00

(54) **VACCIN THERAPEUTIQUE CIBLE CONTRE LA P-GLYCOPROTEINE 170 POUR INHIBER LA RESISTANCE MULTIDROGUES DANS LE TRAITEMENT DES CANCERS**
GEZIELTE THERAPEUTISCHE VAKZINE GEGEN P-GLYCOPROTEIN 170 ZUR HEMMUNG VON ARZNEIMITTEL-MULTIRESISTENZ BEI DER BEHANDLUNG VON KREBS
TARGETED THERAPEUTIC VACCINE AGAINST P-GLYCOPROTEIN 170 FOR INHIBITING MULTI-DRUG RESISTANCE IN THE TREATMENT OF CANCERS

(30) Priorité: 25.07.2003 FR 0309188
(43) Date de publication de la demande: 03.05.2006
(60) Demande divisionnaire: 10185331.5
(73) Titulaire: AC Immune SA, 1015 Lausanne (CH)
(72) Inventeur: TOSI, P., F., F-51100 Reims (FR); MADOULET, Claudine, F-51100 Reims (FR); NICOLAU, Claude, Newton, MA 02459 (US); HICKMAN, David, T., CH-1025 Saint-Sulpice (CH)
(74) Mandataire: Vossius & Partner Patentanwälte Rechtsanwälte mbB
(86) Numéro de dépôt international: PCT/EP2004/008330
(87) Numéro de publication internationale: WO 2005/014036

(56) Documents cités:
- WO-A-94/10198
- TOSI P-F ET AL: "Immune response against the murine mdr1 protein induced by vaccination with sythetic lipopeptides in liposomes" BIOCHEMICAL AND BIOPHYSICAL RESEARCH COMMUNICATIONS, vol. 212, no. 2, 17 juillet 1995 (1995-07-17), pages 494-500, XP002034273 ISSN: 0006-291X cité dans la demande
- MECHETNER E B & RONINSON I B: "Efficient inhibition of P-glycoprotein-mediated multidrug resistance with a monoclonal antibody" PROCEEDINGS OF THE NATIONAL ACADEMY OF SCIENCES OF USA, vol. 89, 1 juillet 1992 (1992-07-01), pages 5824-5828, XP002060367 ISSN: 0027-8424 cité dans la demande
- TSURUO T: "Circumvention of drug resistance with calcium channel blokers and monoclonal antibodies" CANCER TREATMENT AND RESEARCH, vol. 48, 1989, pages 73-95, XP001190802 ISSN: 0927-3042 cité dans la demande
- CHEN C-J ET AL: "Internal duplication and homology with bacterial transport proteins in the mdr1 (P-glycoprotein) gene from multidrug-resistant human cells" CELL, vol. 47, no. 3, 7 novembre 1986 (1986-11-07), pages 381-389, XP000603669 ISSN: 0092-8674
- PAWLAK-ROBLIN C ET AL: "Inhibition of multidrug resistance by immunisation with synthetic P-glycoprotein-derived peptides" EUROPEAN JOURNAL OF CANCER, vol. 40, no. 4, mars 2004 (2004-03), pages 606-613, XP004488461 ISSN: 0959-8049
- ROBERTS M.J. ET AL.: "Chemistry for peptide and protein PEGylation", ADVANCED DRUG DELIVERY REVIEWS, vol. 54, 2002, pages 459-476,

## Description

La présente invention s'inscrit dans le cadre de la recherche et de la mise au point de nouveaux agents utiles pour traiter la résistance multidrogues (résistance pléïotropique ou résistance multidrogues) apparaissant chez certains patients lors du traitement de cancers.

L'invention relate en particulier des agents utilisables pour induire une réponse immunitaire chez des patients souffrant de résistance multidrogues en cours de traitement d'un cancer, dans le but d'atténuer, voire de réverser cette résistance. L'invention concerne aussi de tels agents pour prévenir l'apparition de résistance multidrogues.

L'invention concerne à cet égard une composition comprenant d'une part un véhicule et d'autre part des conjugués formés par liaison covalente entre une région peptidique et des molécules d'acide gras de chaîne carbonée comprise entre C12 et C24, ladite partie peptidique étant dérivée d'au moins l'une des boucles extracellulaires de la protéine P-170. Cette composition immunogène, administrée dans des conditions appropriées permet l'induction d'anticorps anti-P-170.

L'invention concerne également des procédés d'immunisation utilisant les compositions précédentes à base de conjugués associés à un véhicule pharmaceutiquement acceptable, par exemple des liposomes. L'invention concerne en particulier des procédés d'immunisation dont la mise en oeuvre précède, est concomitante ou suit une étape de traitement chimiothérapeutique administré à un patient.

L'invention concerne enfin l'utilisation de la composition selon l'invention pour le traitement *in vivo* de la résistance multidrogues, apparaissant chez un patient souffrant d'un cancer traité au moyen de médicaments anticancéreux, ou le cas échéant pour la prévention d'une telle résistance multidrogues.

Le phénomène de résistance multidrogues (MDR) a été mis en évidence à la fin des années 70 sur des lignées de cellules cancéreuses rendues résistantes à des drogues chimiothérapeutiques notamment des drogues utilisées pour le traitement de cancers. La résistance multidrogues se caractérise par une résistance pléïotropique, vis à vis de drogues chimiothérapeutiques utilisées pour le traitement d'un patient, lorsque ces drogues ont des structures et des spécificités différentes. Parmi les drogues capables de sélectionner ou d'induire la résistance pléïotropique de la cellule cancéreuse, on citera la colchicine, l'adriamycine, l'actinomycine, la vincristine, la vinblastine et la mitoxantrone. D'un point de vue phénotypique la résistance multidrogues se caractérise par une diminution de l'accumulation intracellulaire des drogues cytotoxiques, des modifications physiologiques de la cellule et une surexpression dans la membrane cellulaire de la protéine P-glycoprotéine encore appelée protéine P-gp ou encore protéine P-170 (Van der Bliek et al. 1988. Gene 71(2) : 401-411, Thiebaut et al. 1987 Proc. Natl. Acad. Sci. 84(21) : 7735-7738, Endicott et al. 1989 Annu. Rev. Biochem. 58 : 137-171). La protéine P-170 est responsable d'un flux actif des médicaments hors de la cellule (encore appelé l'efflux actif), ce phénomène étant dépendant de la consommation d'ATP. La reconnaissance par la protéine P-170 et l'excrétion hors de la cellule traitée, médiée par la protéine P-170 d'une grande variété de composés chimiques, de structures et de fonctions diverses, demeurent l'un des aspects les plus énigmatiques de la fonction de cette protéine. L'absence de la mise en évidence d'une caractéristique structurale commune entre les drogues faisant l'objet d'une résistance croisée ne permet pas.d'envisager l'élaboration de drogues qui ne seraient pas expulsées, sous l'influence de la protéine P-170.

La résistance multidrogues des tumeurs aux agents de chimiothérapie constitue un problème central en cancérologie médicale. Tandis que des progrès dans les traitements de soutien sont observés, le problème de la résistance aux drogues demeure un obstacle à l'obtention de meilleurs taux de guérison. On constate que les cellules tumorales peuvent ne pas répondre à la chimiothérapie dès le début du traitement. Cette résistance multidrogues *de novo* est malheureusement fréquente dans plusieurs types de tumeurs solides. On a par ailleurs pu observer un phénomène de résistance acquise, se manifestant au niveau de tumeurs qui, au départ, ont répondu à la chimiothérapie et qui développent ensuite, à plus ou moins court terme, une résistance aux traitements.

Pour être plus efficaces, les traitements anticancéreux ont été associés à des agents modulateurs de la résistance multidrogues également nommés agents révertants pouvant bloquer le flux sortant de drogues médié par la protéine P-170, hors de la cellule et ainsi circonvenir la résistance multidrogues. Les agents révertants existants, tels que le vérapamil, la quinine et la ciclosporine, entraînent une toxicité inacceptable pour le patient lorsqu'ils sont utilisés aux doses nécessaires pour inhiber l'activité d'efflux de la protéine P-170. Par exemple, le vérapamil a rapidement montré ses limites dans le traitement de réversion de cancers du fait de l'apparition chez le patient de dysfonctionnements tels que l'hypotension, l'arythmie cardiaque et l'insuffisance cardiaque congestive lors de son administration aux doses curatives qui sont également les doses limites de toxicité (Miller et coll. 1991. J Clin Oncol 9(1) : 17-24).

Des analogues plus récents, tels que le dexvérapamil, le PSC 833 (dérivé de la cyclosporine) et dernièrement le S9788 des Laboratoires Servier, ont fait l'objet d'essais cliniques ayant pour but de vaincre la résistance multidrogues. Cependant, ces nouveaux agents de réversion connaissent des limites d'utilisation comparables à celles reportées pour l'ancienne génération d'agents de réversion. En effet, les essais de traitement de la résistance multidrogues à l'aide du S9788 (6- [4- [2,2-di (4-fluorophenyl)-éthylamino]-1-piperidinyl]-N,N'-di-2-propenyl-1,3,5-triazine-2,4-diamine), dérivé de triazineaminopiperidine, ont caractérisé les limites d'utilisation de ce produit suite à l'apparition de phénomènes de toxicité cardiaque, d'arythmie ventriculaire et de torsade de pointe (Stupp et coll. 1998. Ann Oncol 9(11): 1233-1242). En conséquence, le phénomène de résistance multidrogues est difficilement endigué par les agents de réversion, nouveaux et classiques, du fait de doses de traitement équivalentes aux seuils de toxicité pour le patient devenu réfractaire à la chimiothérapie.

L'immunothérapie, en particulier l'utilisation des anticorps monoclonaux, a aussi été envisagée pour traiter la résistance multidrogues apparaissant chez le patient. Elle a été testée la première fois pour inhiber la formation de tumeurs dans un cancer ovarien à l'aide de l'anticorps monoclonal MRK16 (Tsuruo. 1989. Cancer Treat Res 48 : 73-95). Plus récemment, l'immunothérapie monoclonale dans le traitement de la résistance multidrogues a été approfondie par Mechetner et Roninson (1992. Proc Natl Acad Sci USA vol.89 pp.5824-5828). En effet, des anticorps monoclonaux UIC2 dirigés contre un épitope extracellulaire de la P-glycoprotéine humaine ont été obtenus et testés *in vitro* sur des lignées cellulaires résistantes aux anticancéreux. Il a alors été montré que l'effet inhibiteur, *in vitro,* des anticorps monoclonaux UIC2 est comparable à celui du vérapamil employé aux doses cliniques maximales (3µM). Les anticorps monoclonaux anti-P-170 exercent leur effet par une inhibition de l'activité ATPasique de la protéine P-170 ainsi que par l'inhibition de la liaison des médicaments à la protéine P-170.

Une immunothérapie appropriée, fondée sur l'injection d'anticorps monoclonaux au patient, peut avoir certains avantages dans la mesure où elle peut éliminer les cellules résistantes résiduelles d'une tumeur. Cependant, la méconnaissance de la spécificité, de la toxicité, de l'efficacité ainsi que du mécanisme d'action des anticorps limitent l'utilisation de cette approche pour vaincre la résistance multidrogues due à la surexpression de la protéine P-170. En particulier, ne sont pas maîtrisés dans l'immunothérapie monoclonale les effets secondaires liés aux réactions immunes anti-anticorps de souris ou de lapin ainsi que les difficultés liées au défaut d'humanisation des anticorps monoclonaux.

La présente invention a donc pour but de proposer une stratégie alternative aux traitements déjà disponibles de la résistance multidrogues dans le cancer, en vue de remédier, au moins en partie, aux inconvénients de traitements connus de la résistance multidrogues. L'invention propose à ce titre, une immunothérapie fondée sur l'induction d'auto-anticorps polyclonaux, spécifiques de la glycoprotéine-P (Protéine P-170). Cette immunothérapie est obtenue en utilisant la capacité antigénique, de conjugués comprenant des peptides dérivés d'au moins l'une des boucles extracellulaires de la protéine P-170, à induire des anticorps chez un patient lorsque ces peptides sont présentés et administrés sous une forme permettant ou favorisant l'expression de leur capacité antigénique, et notamment lorsqu'ils sont associés à un véhicule pharmaceutiquement acceptable. En particulier les anticorps sont des auto-anticorps induits contre la P-170 humaine.

L'invention concerne donc en particulier, une composition immunogène comprenant d'une part un véhicule, d'autre part en tant que structure antigénique, des conjugués comprenant tout ou partie des séquences d'acides aminés d'au moins un peptide dérivé d'une boucle extracellulaire de la protéine P-170, chaque peptide étant associé à au moins deux molécules d'acide gras de chaîne carbonée comprise entre C12 et C24 par le biais d'une ou de plusieurs molécules de PEG pour, dans des conditions d'administration appropriées, permettre l'induction d'anticorps anti-P-170.

Les auteurs de la présente invention ont, de manière surprenante et inattendue, montré qu'une augmentation de 77% du temps de survie chez des souris immunisées par le la composition immunogène telle que décrite dans la présente invention et après inoculation de cellules cancéreuses, suivi d'un plan de traitement chimiothérapeutique était observée.

Ces résultats sont très prometteurs puisque les meilleurs résultats publiés obtenus dans le traitement de la résistance multidrogues avec le même modèle de cancer décrivaient une augmentation de survie de 49% chez des souris traitées par une autre substance (Pierré et coll. 1992. Invest New Drug. 10 : 137-148). De plus, Yang et coll. (1999. BBRC. 266 : 167-173) ont observé, avec la même lignée cellulaire, une augmentation de survie de seulement 35% chez des souris traitées par la vincristine et la ciclosporine A.

Le invention concerne donc une composition immunogène pour l'induction d'anticorps anti-P-170 comprenant un véhicule et, en tant que structure antigénique, des conjugués comprenant tout ou partie des séquences d'acides aminés d'au moins un peptide dérivé d'une boucle extracellulaire de la protéine P-170, chaque peptide étant associé à au moins deux molécules d'acide gras de chaîne carbonée comprise entre C12 et C24, caractérisée en ce que le ou lesdits peptides sont associés aux molécules d'acides gras par le biais d'une ou de plusieurs molécules de PEG, en particulier, d'une ou de plusieurs molécules de PEG, de 1 à 8000 monomères, liées aux résidus lysine (K) se trouvant en position N- et C-terminale des séquences d'acides aminés d'au moins un peptide dérivé d'une boucle extracellulaire de la protéine P-170.

Un objet particulier de l'invention concerne ladite composition immunogène selon la présente invention, caractérisée en ce qu'elle permet l'induction d'anticorps anti-P-170 pour la réversion de la résistance multidrogues apparaissant chez un patient atteint d'un cancer.

Un autre objet particulier de l'invention concerne ladite composition immunogène selon la présente invention, caractérisée en ce que les conjugués comprennent tout ou partie des séquences d'acides aminés d'au moins deux boucles extracellulaires de la protéine P-170, en particulier trois boucles extracellulaires de la protéine P-170.

Dans un mode de réalisation spécifique de l'invention lesdits conjugués comprennent tout ou partie des séquences d'acides aminés sélectionnés parmi les peptides dérivés des boucles extracellulaires 1, 4 et 6 de la protéine P-170 humaine, en particulier de peptides dérivés de la boucle 1 de la protéine P-170 humaine correspondant aux trois peptides, 1a, 1b et 1c, résultant de la coupure de ladite boucle 1 extracellulaire au niveau des sites de glycosylation, lesdits conjugués permettant l'induction d'anticorps anti-P-170 humains (anti-hpp).

Dans un autre mode de réalisation spécifique de l'invention lesdits conjugués comprennent tout ou partie des séquences d'acides aminés sélectionnés parmi les peptides dérivés des boucles extracellulaires 1, 2 et 4 de la protéine P-170 murine, lesdits conjugués permettant l'induction d'anticorps anti-P-170 murins (anti-mpp).

En particulier, la présente invention concerne une composition immunogène selon la présente invention, caractérisée en ce que tout ou partie des séquences d'acides aminés des peptides des conjugués sont respectivement choisis parmi les séquences d'acides aminés suivantes :
pour la boucle 1 :
   le peptide 1 SEQ ID NO 4:
      GEMTDIFANAGNLEDLLMSNITNRSDINDTGFFMNLEEDMTRYAYYYS
   ou le peptide 1a SEQ ID NO 5: GEMTDIFANAGNLEDLLMS
   ou le peptide 1b SEQ ID NO 6: NITNRSDINDTGFF
   ou le peptide 1c SEQ ID NO 7: MNLEEDMTRYAYYYS
pour la boucle 4 :
   SEQ ID NO 8: FSRIIGVFTRIDDPETKRQNSNLFS
pour la boucle 6 :
   SEQ ID NO 10: FRFGAYLVAHKLMSFED
et/ou leur combinaison, et/ou les séquences d'acides aminés modifiées, dès lors que les séquences d'acides aminés ainsi modifiées permettent, dans des conditions d'administration appropriées, l'induction d'anticorps anti-P-170.

Un autre objet particulier de l'invention concerne ladite composition immunogène selon la présente invention, caractérisée en ce que tout ou partie des séquences d'acides aminés des peptides des conjugués sont respectivement choisis parmi les séquences d'acides aminés suivantes :
pour la boucle 1 :
   le peptide 1 SEQ ID NO 1:
      GNMTDSFTKAEASILPSITNQSGPNSTLIISNSSLEEE
pour la boucle 2 :
   SEQ ID NO 2: KVLTSFTNKELAYAK
pour la boucle 4 :
   SEQ ID NO 3: SRDDDMETKRQNEN
et/ou leur combinaison, et/ou les séquences d'acides aminés modifiées, dès lors que les séquences d'acides aminés ainsi modifiées permettent, dans des conditions d'administration appropriées, l'induction d'anticorps anti-P-170.

Un autre objet particulier de l'invention concerne ladite composition immunogène selon la présente invention, caractérisée en ce que chaque peptide étant lié de manière covalente à au moins deux chaînes de polyéthylène glycol, en particulier, deux chaînes de polyéthylène glycol couplées chacune a une molécule de phosphatidyl éthanolamine.

L'invention concerne en outre une composition immunogène selon la présente invention, caractérisée en ce que le véhicule choisi pour présenter les conjugués est choisi dans le groupe consistant en des liposomes, des protéines membranaires bactériennes, des protéines Omp d'entérobactéries, des nanoparticules, des miscelles, des particules d'or, des microbilles et des virosomes, en particulier, des liposomes.

Dans un mode de réalisation spécifique de l'invention, les conjugués et les liposomes ont un rapport molaire compris entre 1/10 et 1/1000, de préférence de 1/250.

Dans un autre mode de réalisation spécifique de l'invention, les liposomes sont de préférence obtenus par mélange des phospholipides dimyristoyle phosphatidylcholine (DPMC), glycérol de phosphatidyle de dimynstoyle (DPMG) et cholestérol.

En particulier, le mélange de DPMC, DPMG et cholestérol est dans les proportions 0.9 :0.1 0.7.

Dans un mode de réalisation particulier de l'invention, la composition immunogène selon la présente invention, comprenant en outre au moins un adjuvant, en particulier, un adjuvant choisi dans le groupe consistant en de l'alum du phosphate de calcium, de l'interleukine 1, du monophosphoryl lipide A (MPLA) et/ou des microcapsules de protéines et de polysaccharides, en particulier, l'alum.

La présente invention concerne en outre une composition immunogène selon la présente invention, pour l'utilisation dans le traitement et/ou la prévention d'une résistance multidrogues apparaissant chez un patient atteint d'un cancer, en particulier un cancer qui atteint le rein, le foie, le colon, l'intestin, la prostate, le sein, la vessie, le cerveau, le sang (leucémie) et/ou les tissus médulaires (myélome).

En particulier, le cancer est une tumeur solide exprimant le gène MDR1 codant pour la protéine P-170 humaine

La présente invention concerne en outre une composition immunogène selon la présente invention, pour l'utilisation dans le traitement d'une résistance multidrogues apparaissant chez un patient atteint d'un cancer traité au moyen de médicaments anticancéreux.

En particulier, le traitement comprend une première administration, et une administration de rappel de ladite composition.

Dans un mode de réalisation particulier de l'invention, la méthode d'immunisation est mise en oeuvre de manière concomitante ou précédent un traitement anticancéreux.

La présente invention est illustrée sans pour autant être limitée, par les figures suivantes :

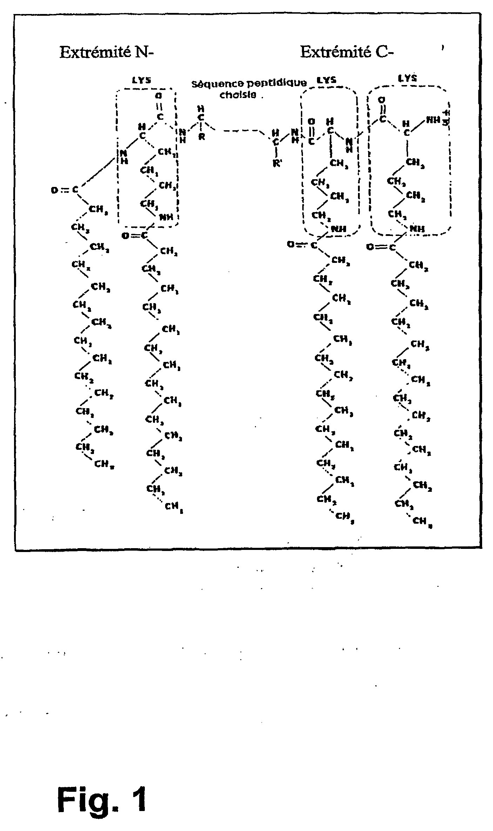

- Figure 1: représentation des peptides synthétiques correspondant aux fragments extracellulaires de la protéine P-170 de souris, couplés à quatre molécules d'acide palmitique (C16) par molécule de peptide.

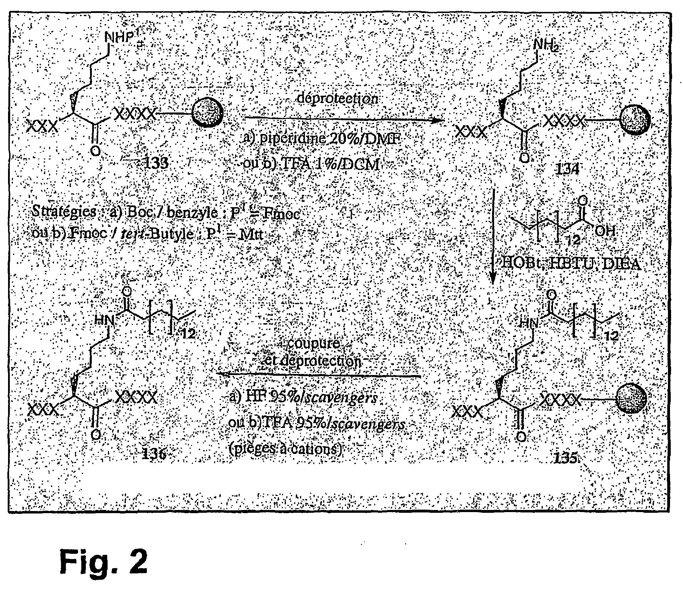

- Figure 2: schématisation de la stratégie de synthèse Boc/benzyle des peptides sur support solide.

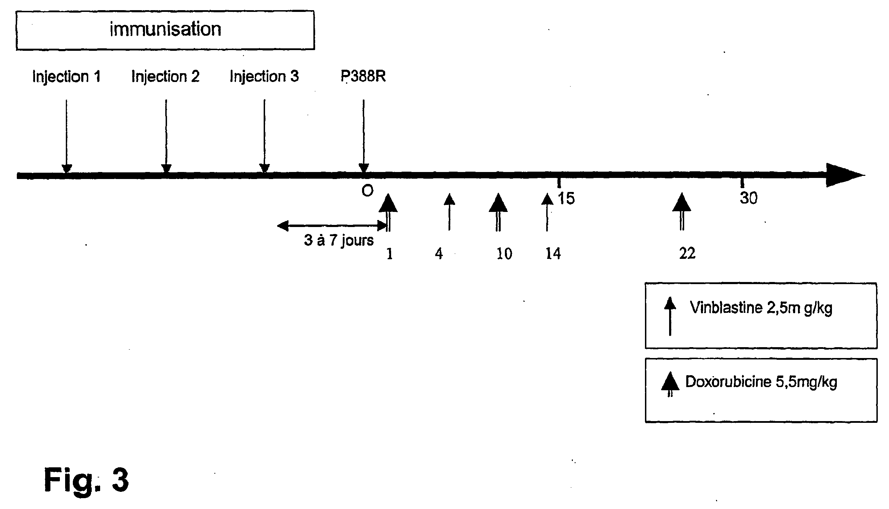

- Figure 3 : protocole de chimiothérapie après immunisation et injection de cellules P388R chez la souris.

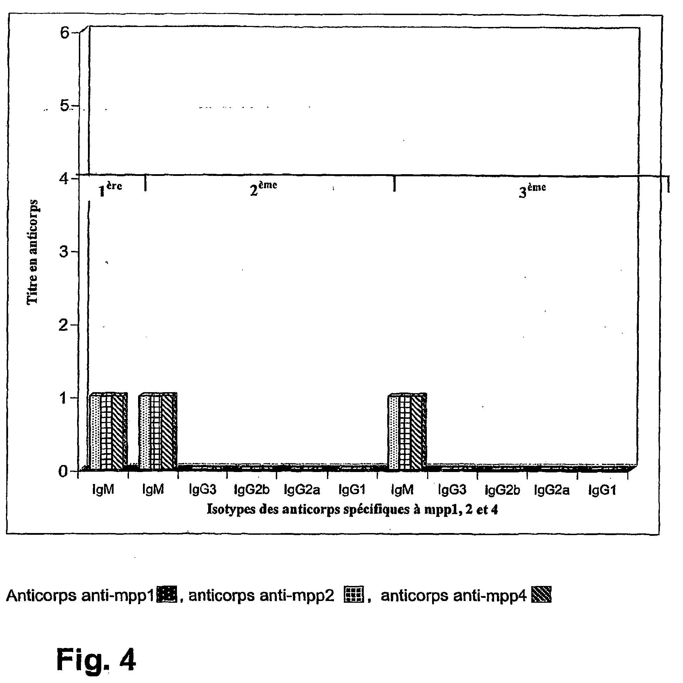

- Figure 4 : représentation du titre en anticorps en fonction du temps d'immunisation (1^{ère}, 2^{ème}, 3^{ème} injection) dans les sérums de souris immunisées par Lp2. Les anticorps anti-mpp1, mpp2, mpp4 ont été quantifiés et les différents isotypes détectés en utilisant des anticorps secondaires anti-murins spécifiques Ig (M, G3, G2a, G2b, G1) respectivement. Chaque histogramme représente la moyenne des valeurs obtenues pour 5 sérums de souris saignées 12 jours après une immunisation. Une unité correspond à 0,2µg lg/ml.

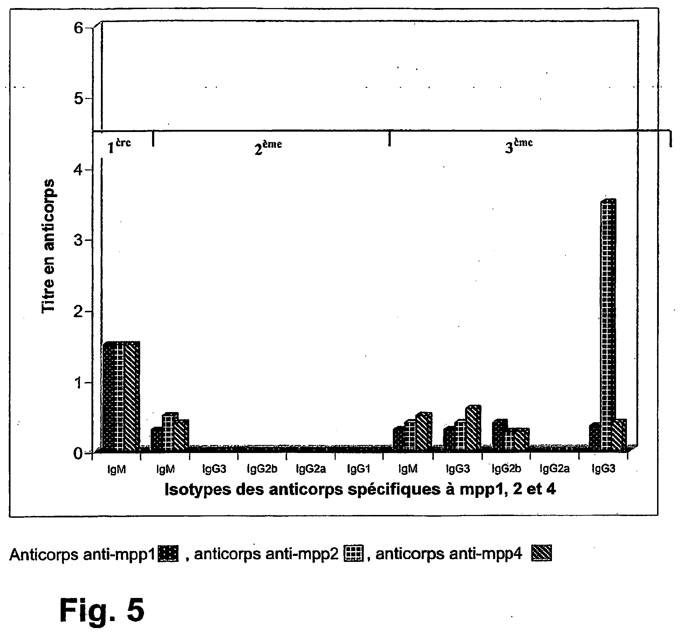

- Figure 5: représentation du titre en anticorps en fonction du temps d'immunisation (1^{ère}, 2^{ème}, 3^{ème} injection) dans les sérums de souris immunisées par Lp4. Les anticorps anti-mpp1, mpp2, mpp4 ont été quantifiés et les différents isotypes détectés en utilisant des anticorps secondaires anti-murins spécifiques Ig (M, G3, G2a, G2b, G1) respectivement. Chaque histogramme représente la moyenne des valeurs obtenues pour 5 sérums de souris saignées 12 jours après une immunisation. Une unité correspond à 0,2µg lg/ml.

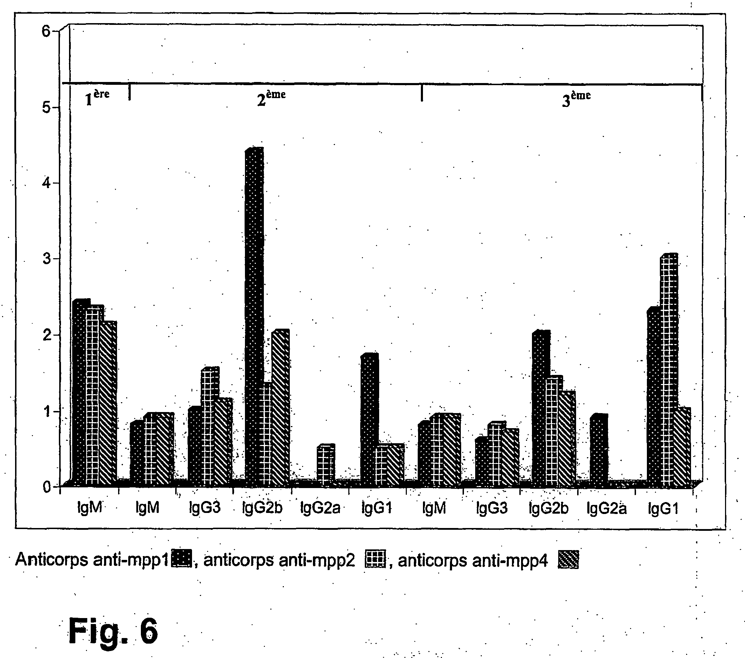

- Figure 6 : représentation du titre en anticorps en fonction du temps d'immunisation (1^{ère}, 2^{ème}, 3^{ème} injection) dans les sérums de souris immunisées par Lp3. Les anticorps anti-mpp1, mpp2, mpp4 ont été quantifiés et les différents isotypes détectés en utilisant des anticorps secondaires anti-murins spécifiques Ig (M, G3, G2a, G2b, G1) respectivement. Chaque histogramme représente la moyenne des valeurs obtenues pour 5 sérums de souris saignées 12 jours après une immunisation. Une unité correspond à 0,2µg lg/ml.

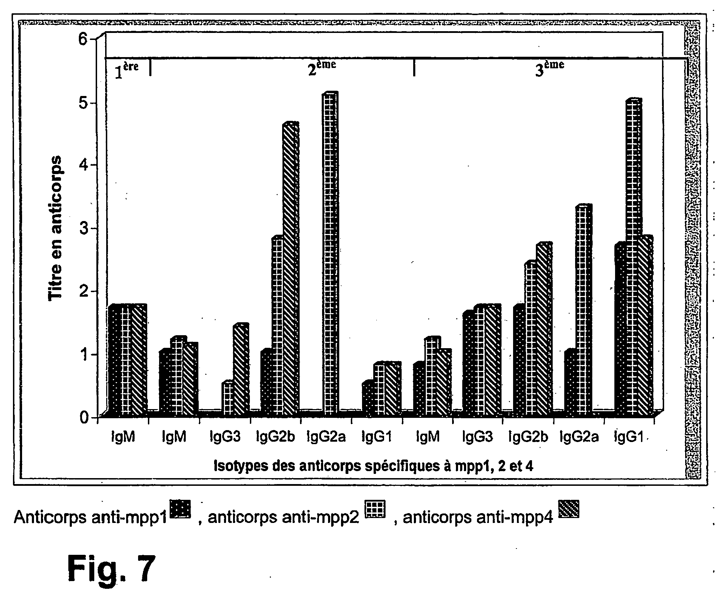

- Figure 7 : représentation du titre en anticorps en fonction du temps d'immunisation (1^{ère}, 2^{ème}, 3^{éme} injection) dans les sérums de souris immunisées par Lp1. Les anticorps anti-mpp1, mpp2, mpp4 ont été quantifiés et les différents isotypes détectés en utilisant des anticorps secondaires anti-murins spécifiques Ig (M, G3, G2a, G2b, G1) respectivement. Chaque histogramme représente la moyenne des valeurs obtenues pour 5 sérums de souris saignées 12 jours après une immunisation. Une unité correspond à 0,2µg lg/ml.

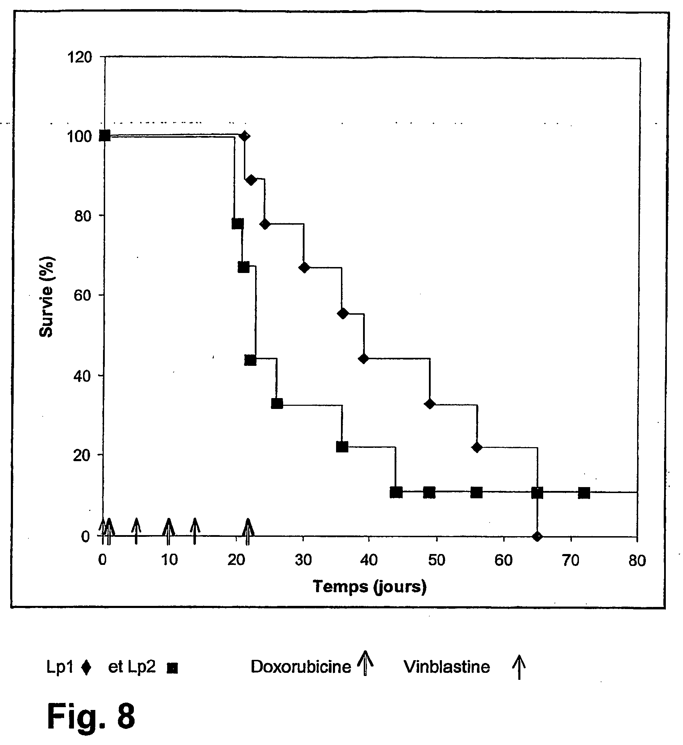

- Figure 8 : Temps de survie des souris immunisées par Lp1 et Lp2. Au temps 0, 10⁶ P388R cellules chimiorésistantes ont été inoculées. Aux jours 1, 10 et 22, 5,5mg/kg de doxorubicine et aux jours 4 et 14, 2'5mg/kg de vinblastine ont été injectés.

La glycoprotéine-P ou protéine P-170 est une phospho-glycoprotéine membranaire de 170 kDa identifiée par Juliano et Ling (1976. Biochim Biophys Acta 455(1) : 152-162). La protéine P-170 murine est constituée de 1276 acides amines formant deux moitiés équivalentes. Les domaines hydrophobes de la molécule sont numérotés de 1 à 12 et sont impliqués dans le flux sortant de drogues chimiothérapeutiques. Les boucles extracellulaires 1, 2 et 4 de la protéine P-170 murine ont été retenues pour leur topologie extracellulaire prononcée préjugeant de leur caractère antigénique. De manière équivalente, la protéine P-170 humaine (Chen et al. 1986 Cell. 47(3): 381-389) est une protéine de 1280 acides aminés constituée de deux domaines homologues comprenant chacun six domaines en hélice transmembranaires ainsi qu'un site de liaison nucléotidique. Les régions hydrophobes de ces domaines transmembranaires forment des boucles extracellulaires considérées comme les fragments de reconnaissance de la protéine P-170 depuis l'extérieur de la cellule. Les boucles extracellulaires 1, 4 et 6 de la protéine P-170 humaine ont aussi été retenues comme ayant un pouvoir antigénique élevé du fait de leurs situations extracellulaires particulièrement marquées.

L'immunothérapie mise en oeuvre dans le cadre de l'invention complète la thérapie chimique du cancer pour permettre aux effets traitants des drogues chimiothérapeutiques de se manifester.

Conformément à l'acception usuelle dans le cadre de l'invention, le « cancer » se définit par deux caractéristiques principales : la croissance et la prolifération cellulaire non régulées par des signaux externes et la capacité d'envahir les tissus ainsi que, le cas échéant, la capacité de former des métastases en colonisant des sites à distance.

Ces caractéristiques sont la conséquence des propriétés intrinsèques des cellules cancéreuses, c'est-à-dire de leur instabilité caryotypique et génomique, de leur prolifération incontrôlée, de leur pouvoir métastatique s'accompagnant de l'acquisition de nouveaux phénotypes ainsi que de l'activation et de la dérépression d'oncogènes dans lesdites cellules cancéreuses. On entend donc par « cancer » dans le cadre de la présente invention toute phase de la croissance ou de la prolifération cellulaire ayant les caractéristiques ci-dessus, évoluant notamment vers le développement de tumeurs primaires et/ou de tumeurs métastatiques (tumeurs secondaires).

De plus, au sens de la présente invention, on entend par « traitement de la résistance multidrogues » l'ensemble des soins médicaux destinés à combattre la résistance multidrogues pour en limiter les conséquences, éviter la mort et de préférence rétablir une sensibilité aux médicaments anticancéreux. A ce titre, l'objectif du traitement de la résistance multidrogues est idéalement à visée curative de la résistance multidrogues, en induisant une réversion complète vers un phénotype cellulaire sensible à la chimiothérapie. Cette réversion peut néanmoins être partielle : dès lors, le traitement de la résistance multidrogues va s'avérer palliatif permettant une rémission prolongée du patient. Le traitement de la résistance multidrogues se caractérise également par son pouvoir prophylactique permettant de prévenir l'apparition de résistances multidrogues. *de novo* ou acquise, chez le patient.

L'invention concerne donc en particulier, une composition immunogène comprenant d'une part un véhicule, d'autre part en tant que structure antigénique, des conjugués comprenant tout ou partie des séquences d'acides aminés d'au moins un peptide dérivé d'une boucle extracellulaire de la protéine P-170, chaque peptide étant associé à au moins deux molécules d'acide gras de chaîne carbonée comprise entre C12 et C24 par le blais d'une ou de plusieurs molécules de PEG pour, dans des conditions d'administration appropriées, permettre l'induction d'anticorps anti-P-170.

Dans le cadre de la présente invention, la composition immunogène permet également l'induction d'anticorps anti-P-170 pour la réversion de la résistance multidrogues apparaissant chez un patient atteint d'un cancer.

Dans un mode de réalisation préféré, les conjugués comprennent tout ou partie des séquences d'acides aminés d'au moins deux boucles, de préférence d'au moins trois boucles extracellulaires de la protéine P-170.

L'expression « conjugué » correspond, selon l'invention, à un réactif formé par liaison covalente entre des molécules d'acides gras de chaîne carbonée comprise entre C12 et C24 et des séquences d'acides aminés telles que décrites dans la présente invention, pour former une molécule mixte lipido-peptidique. La séquence peptidique, obtenue notamment par synthèse en phase solide, est couplée de manière covalente aux molécules d'acides gras constituant la région lipidique de la molécule.

Au sens de la présente invention, le terme « peptide dérivé » de la protéine P-170 désigne tout ou partie de la séquence d'acides aminés composant chaque boucle extracellulaire de la protéine P-170, dès lors que ledit « peptide dérivé » possède au moins un épitope de la boucle extracellulaire dont il dérive. Un peptide dérivé d'une boucle extracellulaire a avantageusement entre 5 et 50 résidus d'acides aminés, de préférence entre 5 et 40, ou entre 10 et 30, avantageusement entre 10 et 25 résidus. Entrent dans le cadre de cette définition, (1) des peptides dont la séquence d'acides aminés est identique à la séquence correspondante de la boucle extracellulaire dont Ils dérivent ou (2) des peptides dont la séquence d'acides aminés est modifiée par rapport à la séquence de la boucle extracellulaire dont ils dérivent, ladite modification pouvant consister en une mutation ponctuelle par insenlion, délétion ou substitution, en particulier substitution conservative, d'un ou plusieurs résidus dès lors que le peptide ainsi constitué reste porteur d'épitope, de la protéine P-170. En particulier une mutation acceptable est une mutation qui ne perturbe pas la conformation du peptide modifié lorsqu'il est inclus dans la composition de l'invention. Ceci peut être vérifié par la capacité du peptide modifié à Induire des anticorps lorsqu'il est formulé dans une composition conformément à l'invention.

Les peptides de l'invention sont obtenus de préférence par synthèse chimique, en particulier par mise en oeuvre des méthodes décrites ci-après.

Dans le contexte de l'invention, l'expression « boucle extracellulaire » de la glycoprotéine-P (ou Protéine P-170) désigne chaque séquence d'acides aminés de la protéine P-170 ayant une topologie extracellulaire ou une relation significative avec le milieu extracellulaire pour pouvoir être sélectionnée en tant que séquence porteuse d'épitope de cette protéine.

Les séquences d'acides aminés formant les peptides synthétiques utilisés dans le cadre de l'invention peuvent être en tant que telles antigéniques, ou être antigéniques dès lors qu'elles sont présentées dans une conformation telle qu'elles conservent la conformation de la séquence d'acides aminés qui leur correspond dans la boucle extracellulaire dont dérive le peptide, ou une conformation suffisamment similaire pour conférer au peptide formé une capacité à induire la production d'anticorps dans des conditions d'administration appropriées. La capacité à induire la production d'anticorps pourra par exemple être vérifiée chez la souris immunisée avec les peptides préparés dans le cadre de l'invention ou par tout autre moyen connu. Ainsi les peptides de l'invention ont avantageusement, dans la composition immunogène, une conformation tridimensionnelle telle qu'elle reproduit la conformation de la partie de boucle extracellulaire dont dérive le peptide, ou qu'elle est suffisamment similaire à cette dernière, pour conférer à la composition formée, sa capacité immunogène. La présentation appropriée des peptides résulte avantageusement de leur association avec les autres constituants de la composition immunogène.

Cette faculté à induire la production d'anticorps est en particulier obtenue lorsque les peptides de l'invention sont des peptides synthétisés et modifiés pour se présenter sous la forme de conjugués, associés à un véhicule approprié, en particulier des liposomes.

Les conjugués selon l'invention correspondent aux structures antigéniques transportées par le véhicule dans la composition immunogène. Dans le contexte de l'invention, l'expression « structure antigénique » désigne des molécules capables de réagir avec des anticorps pour former des complexes antigène/anticorps. Ladite « structure antigénique » de la composition immunogène a, ou non, en tant que telle la capacité d'induire un phénomène d'immunogénicité correspondant à la formation d'anticorps spécifiques d'un antigène donné.

A titre d'exemples, ces conjugués, comprennent des peptides dérivés de la boucle extracellulaire 1 (mpp1), et d'au moins une des boucles extracellulaires 2 (mpp2) ou 4 (mpp4) de la protéine P-170 murine, caractérisées par leur séquence, leur masse moléculaire et leur pureté après synthèse selon le protocole expérimental décrit ci-après dans le tableau I.

**Tableau I :**

| Nom des conjugués | Séquence en acides aminés | Masse moléculaire calculée | Masse moléculaire observée | Pureté (%) |
|---|---|---|---|---|
| mpp1 SEQ ID NI 1 | K-G-GNMTDSFTKAEASILPSITNQSGPNSTLIISNSSLEEE-G-K-K-NH₂ | 5436 | 5437 | 91.6 |
| mpp2 SEQ ID NO 2 | K-G-KVLTSFTNKELAYAK-G-K-K-NH₂ | 3293 | 3293 | 93.8 |
| mpp4 SEQ ID NO 3 | K-G-SRDDDMETKRQNEN-G-K-K-NH₂ | 3190 | 3188 | 95.3 |

Les masses moléculaires calculées et observées des peptides dérivés des boucles extracellulaires 1, 2 et 4 de la protéine P-170 murine ont été obtenues par analyse par spectrométrie de masse soit :
- [M+H+] pour la masse moléculaire calculée,
- MALDI-TOF et PDMS-TOF pour la masse moléculaire mesurée, i.e.
   (Matrix Assisted Lazer Desorption Ionization - Time of Flight)
   (Plasma Desorption Mass Spectrometry - Time of Flight).

De plus les séquences en acides aminés sont également analysées par hydrolyse des aliquots dans HCl 6N/phénol à 110°C afin de vérifier le nombre d'acides aminés attendu et obtenu (attendu/obtenu) :
mpp 1, A (2/1.5), D (5/4.4), E (5/6.6), F (1/0.6), G (4/3.4), 1 (4/3.6), K (4/4.0), L(3/3.5), M (1/0.7), P (2/1.8), T (4/3.0), S (8/7.4).
mpp 2, A (2/2.3), D (1/1.2), E (2/2.4), F (1/1.0), G (2/2.1), K (6/6.0), L (2/2.1), S (1/0.9), V (1/1.0), Y (1/1.0).
mpp 4, D (5/5.0), E (3/3.1), G (2/2.0), K (4/3.7), S (1/0.9), R (2/1.9).

Selon un mode de réalisation particulièrement avantageux de l'invention, les conjugués comprennent des peptides dérivés de la boucle extracellulaire 1 (hpp1) (hpp : human palmitoyl peptide) et d'au moins une des boucles extracellulaires 4 (hpp4) et 6 (hpp6) de la protéine P-170 humaine. Les peptides des conjugués comprennent donc des séquences d'acides aminés dérivées des boucles extracellulaires 1(hpp1) et 4 (hpp4) ou des boucles extracellulaires 1 (hpp1) et 6 (hpp6).

De préférence, les conjugués de la composition immunogène comprennent des peptides dérivés des trois boucles 1 (hpp1), 4 (hpp4) et 6 (hpp6) de la protéine P-170 humaine.

Etant donné la longueur de 47 acides aminés de la boucle 1 de la protéine P-170, des peptides dérivés de cette boucle peuvent résulter de la division en trois fragments de ladite boucle 1, obtenus par coupure au niveau des sites de glycosylation. Les trois peptides dérivés donnent lieu aux trois conjugués suivants hpp1a, hpp1b et hpp1c dont on peut réaliser la synthèse. D'autres peptides peuvent être des fragments de ces trois peptides, contenant un ou plusieurs épitopes. Dès lors, les conjugués selon la présente invention comprennent donc tout ou partie des peptides dérivés de la boucle extracellulaire 1 de la protéine P-170 humaine correspondant aux trois peptides résultant de la coupure de ladite boucle 1 au niveau des sites de glycosylation.

Les séquences peptidiques des conjugués sont utilisables en tant que telles pour la préparation de compositions immunogènes selon l'invention en particulier en association avec des liposomes. Les peptides de ces conjugués sont respectivement choisis parmi les séquences d'acides aminés suivantes :
- pour la boucle 1 (SEQ ID NO 4):
- pour la boucle 1 a (SEQ ID NO 5): GEMTDIFANAGNLEDLLMS
- pour la boucle 1b (SEQ ID NO 6) : NITNRSDINDTGFF
- pour la boucle 1c (SEQ ID NO 7): MNLEEDMTRYAYYYS
- pour la boucle 4 (SEQ ID NO 8): FSRIIGVFTRIDDPETKRQNSNLFS
- pour la boucle 4a (SEQ ID NO 9) : FTRIDDPETKRQNSNLFS
- pour la boucle 6 (SEQ ID NO 10): FRFGAYLVAHKLMSFED et/ou leur combinaison.

Pour la boucle extracellulaire 1, on utilisera avantageusement les trois peptides 1a, 1b et 1c dans une même composition. Alternativement les peptides 1a et 1b ou 1a et 1c ou 1b et 1c seront mis en oeuvre.

Le tableau II récapitule les séquences en acides aminés des conjugués correspondant aux boucles extracellulaires 1, 4 et 6 de la protéine P-170 humaine. Les séquences en acides aminés correspondant aux séquences des peptides dérivés sont en grandes lettres alors que les petites lettres correspondent aux acides aminés ajoutés sur lesquels les molécules d'acide gras sont couplées.

La séquence, la masse moléculaire et la pureté des peptides décrits dans le tableau II peuvent être contrôlées par les techniques décrites ci-dessus pour les peptides du Tableau I.

Un peptide dérivé d'une boucle extracellulaire a avantageusement une pureté égale ou supérieure à 90%, avantageusement comprise entre 91 % et 98%, mesurée en chromatographie HPLC après la synthèse.

**Tableau II :**

| Nom des conjugués | Séquence en acides aminés de la P-170 humaine |
|---|---|
| Hpp1 (SEQ ID NO 11) | |
| Hpp1a (SEQ ID NO 12) | K-G-GEMTDIFANAGNLEDLLMS-G-K-K-NH₂ |
| Hpp1b (SEQ ID NO 13) | K-G-NITNRSDINDTGFF-G-K-K-NH₂ |
| Hpp1c (SEQ ID NO 14) | K-G-MNLEEDMTRYAYYYS-G-K-K-NH₂ |
| Hpp4 (SEQ ID NO 15) | K-G-FSRIIGVFTRIDDPETKRQNSNLFS-G-K-K-NK₂ |
| Hpp4a (SEQ ID NO 16) | K-G-FTRIDDPETKRQNSNLFS-G-K-K-NH₂ |
| Hpp6 (SEQ ID NO 17) | K-G-FRFGAYLVAHKLMSFED-G-K-K-NH₂ |

Dans le cadre de la présente invention, les séquences d'acides aminés correspondant aux séquences des boucles extracellulaires (représentées en grandes lettres) peuvent être allongées notamment à leur(s) extrémité(s) au moyen de résidus d'acides aminés (représentés dans les peptides illustrés ci-après en petites lettres majuscules) sur lesquels les résidus d'acides gras sont couplés. Différentes séquences de conjugués de l'invention couplant séquences d'acides aminés et molécules d'acide gras sont représentées sous cette forme dans les tableaux qui suivent.

Avantageusement, les molécules d'acide gras de chaîne carbonée C12, C13, C14, C15, C16, C17, C18, C19, C20, C21, C22, C23 ou C24 sont de préférence des molécules d'acide palmitique en C16. Les chaînes carbonées des molécules d'acide gras comprises entre C12 et C24 sont linéaires ou ramifiées. De préférence, les molécules d'acide gras ont des chaînes carbonées linéaires. En revanche, les molécules d'acide gras ne peuvent être ni monoinsaturées ni polyinsaturées en raison d'incompatibilité réactionnelle lors de la synthèse peptidique en particulier durant la dernière étape de déprotection en présence d'acide fort.

De façon préférée, chaque conjugué comprend au moins quatre molécules d'acide gras de chaîne carbonée comprise entre C12 et C24, les molécules d'acides gras étant, de préférence également réparties aux extrémités N- et C- terminales des peptides. Selon les acides gras d'autres répartitions peuvent être envisagées y compris dans l'intérieur de la séquence d'acides aminés. Ces peptides sont également couplés de façon covalente aux molécules d'acide gras.

Préférentiellement, les peptides, dans les conjugués, sont chacun couplés à quatre molécules d'acide palmitique, ils sont donc tétrapalmitoylés.

De préférence deux molécules d'acide palmitique sont couplées à l'extrémité N-terminale et deux molécules d'acide palmitique sont couplées à l'extrémité C-terminale du peptide.

De façon préférée et comme illustrée au tableau II, les séquences d'acides aminés des boucles extracellulaires des peptides des conjugués sont allongées en.position N- et/ou C-terminale par un ou plusieurs résidus d'acides aminés pour permettre l'association aux molécules d'acide gras dé chaîne carbonée comprise entre C12 et C24.

L'association desdits peptides aux acides gras peut être réalisée exclusivement en position N-terminale ou alternativement exclusivement en position C-terminale. Avantageusement, dans les conjugués, l'association des peptides aux acides gras est réalisée en position N-terminale et C-terminale des séquences peptidiques, en particulier pour donner des séquences tétrapalmitoylées.

Alternativement ou cumulativement, on peut envisager d'associer des acides gras à des résidus internes dans la séquence peptidique.

Les conjugués tels que définis dans la présente invention peuvent également comprendre en outre une ou plusieurs molécules de PEG.

La « pégylation », c'est à dire le procédé qui consiste à coupler de manière covalente une molécule de PEG à un peptide, pour augmenter l'immunogénicité / le caractère immunogène d'un peptide utilisé comme antigène est une technique bien connue de l'homme du métier (2002. Adv Drug Deliv Rev. 54(4): 459-476).

Ce procédé permet, notamment, d'augmenter l'accessibilité de la séquence peptidique et par ce biais la présentation de l'antigène.

De manière générale, plus la séquence peptidique est longue, plus le nombre de molécules de PEG sera élevé.

Avantageusement, la ou les molécules de PEG, de 1 à 8000, sont liées aux résidus lysine (K) se trouvant en position N- et/ou C-terminale des séquences d'acides aminés des boucles extracellulaire des peptides des conjugués tels que décrits dans la présente invention. Chaque peptide étant lié de manière covalente à au moins deux molécules d'acide gras de chaîne carbonée comprise entre C12 et C24 ou à au moins deux chaines de polyéthylèneglycol, (1-8000) qui sont couplées chacune à une molécule de phosphatidyléthanolamine afin de permettre dee reconstituer ces complexes antigéniques dans la bicouche lipidique des liposomes. Dans des conditions d'administration appropriées, ces antigènes reconstitues dans des liposomes-adjuvants, l'induction d'anticorps anti-P-170.

Dans le contexte de l'invention, l'expression « véhicule » d'une composition immunogène désigne tout agent assurant le transport des structures antigéniques dans le système immunitaire. En particulier, un véhicule conforme à l'invention pourra être constitué de liposomes, de protéines membranaires bactériennes telles que les OMPC de *Neissera meningitidis,* TraT *d'Escherichia coli,* de protéines Omp d'entérobactéries, de nanoparticules, de miscelles, de particules d'or, de microbilles et de virosomes.

Pour former les compositions immunogènes de l'invention à partir des susdits conjugués, on choisit avantageusement les liposomes comme véhicule pour présenter les conjugués dans la composition de la présente invention.

Avantageusement, lesdits conjugués sont présents à la surface des liposomes.

On entend par « liposomes » au sens de la présente invention une particule sphérique artificielle constituée d'une ou plusieurs couches de phospholipides, assurant la présentation aux cellules du système immunitaire des peptides porteurs des épitopes et dérivés des boucles extracellulaires de la glycoprotéine-P (P-170).

La composition selon la présente invention comprend avantageusement les conjugués et les liposomes dans un rapport molaire compris entre 1/10 et 1/1000, de préférence entre 1/50 et 1/500, avantageusement dans un rapport molaire de 1/250.

Avantageusement, les liposomes sont préparés en mélangeant le dimyristoylphosphatidylcholine (DPMC), le dimyristoylphosphatidylglycerol (DMPG) et le cholestérol dans un rapport molaire respectivement de 0.9 : 0.1: 0.7. Les produits utilisés supra sont de préférence d'origine synthétique afin d'éviter les possibilités de contamination par des endotoxines, prions ou virus. Par exemple, les phospholipides DMPC et DMPG sont d'origine synthétique (Avanti Polar Lipids USA) et le cholestérol, de pureté à 98%, est d'origine animale. Le monophosphoryl lipide A (MPLA), également d'origine synthétique, et connu pour accroître la réponse immunitaire (Fries et coll. 1992. Proc Natl Acad Sci 89(1) : 358-362) a été ajouté et testé à des liposomes dans une concentration de 40µg par µmole de phospholipides.

La composition selon la présente invention comprend en outre au moins un adjuvant. Le terme « adjuvant » est utilisé dans le contexte de la présente invention pour désigner un produit permettant un stimuli non spécifique des réponses immunes et augmentant l'antigénicité des structures antigéniques L'adjuvant agirait en mobilisant les cellules phagocytaires vers le lieu de dépôt des structures antigéniques et en assurant un relargage plus lent des antigènes, ce qui prolonge la stimulation du système immunitaire.

En particulier, les adjuvants utilisés dans la présente composition immunogène sont choisis dans le groupe consistant en l'alum, phosphate de calcium, interleukine 1, monophosphoryl lipide A (MPLA) et/ou microcapsules de protéines et de polysaccharides. Avantageusement, l'alum est l'adjuvant utilisé dans le cadre de la présente invention.

De telles compositions immunogènes sont préparées sous la forme de solutions liquides, ou de suspensions injectables, ou encore sous une forme solide adaptée à la mise en solution préalable à l'injection dans le cadre, par exemple, d'un kit d'exploitation de la présente composition tel que décrit plus loin.

L'invention porte également sur des peptides dérivés d'au moins une boucle extracellulaire de la protéine P-170 et permettant l'induction d'anticorps anti-P-170 lorsqu'ils sont utilisés dans les compositions immunogènes telles que décrites. Ces peptides sont respectivement choisis parmi les séquences d'acides aminés suivantes :
SEQ ID NO 1, SEQ ID NO 2, SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16 et SEQ ID NO 17.

Egalement comprises dans la présente invention sont les séquences d'acides nucléiqes, ADN ou ARN, codant pour les peptides tels que décrits supra.

La présente invention concerne non seulement, la composition immunogène considérée en tant que telle et définie supra, mais également une méthode de préparation de cette composition.

Les résultats expérimentaux ci-après montrent que l'association de peptides particuliers à uniquement deux molécules d'acide palmitique peut ne pas conduire à l'expression d'une réponse immunogène. Ceci traduit la nécessité de sélectionner les acides gras pour conférer aux conjugués formés, la conformation nécessaire à l'induction d'une réponse immunitaire.

Une attention particulière est également apportée à la synthèse des conjugués selon l'invention et en particulier à leur région peptidique. En effet, les travaux précédents de Tosi et coll. (1995) ont mis en évidence que la séquence peptidique de mpp1, qui est le plus long des conjugués avec 43 acides aminés, ne permettait pas l'obtention d'une réponse immunitaire. En l'absence d'explications et d'hypothèses de travail sur ce constat, les inventeurs ont révélé, dans le cadre de la présente invention, le rôle essentiel d'une synthèse peptidique très précise. Les inventeurs ont donc développé et montré qu'une méthode de synthèse améliorée permettant une production plus efficace de certains acides aminés en évitant leur implication dans des réactions de terminaison précoce permettait d'obtenir en particulier une séquence mpp1 immunoréactive. Les conjugués selon l'invention peuvent donc obtenus sous une forme appropriée, pour réaliser la composition immunogène de l'invention par une synthèse sur support solide selon la stratégie Boc/benzyle.

En l'espèce comme décrit dans la partie exemples, les peptides correspondant aux séquences des boucles extracellulaires 1, 2 et 4 de la P-170 murine ont donc été synthétisés par un synthétiseur de peptide « Applied Biosystems 430A » utilisant le tertiobutyloxycarbonyl/benzyl ou stratégie Boc/benzyle et *in situ* une activation par le (N-[(1H-benzotriazol-1-yl)(diméthylamino)méthylène]-N-méthylméthanaminium hexafluorophosphateN-oxyde.

La synthèse des peptides peut être réalisée sur un synthétiseur de peptides, par exemple un synthétiseur « Applied Biosystems 430A » en utilisant le (13, 14) tertiobutyloxycarbonyl/benzyl et *in situ* une activation par le (N-[(1H-benzotriazol-1-yl)(diméthylamino)méthylène]-N- méthylméthanaminium hexafluorophosphate N-oxyde (Schölzer M. et al Science 1992, 256 (5054) : 221-225).

Selon cette méthode, la synthèse des conjugués repose sur la synthèse des séquences d'acides aminés des boulces extracellulaires des peptides des conjugués sur support solide selon la stratégie Boc/benzyle, puis un alongement en position N- et/ou C-terminale par un ou plusieurs résidus d'acides aminés pour permettre d'association aux molécules d'acide gras de chaine carbonée comprise entre C12 et C24, suivi d'une étape de déprotection des fonctions aminés des lysines N et C-terminales afin de les coupler avec les acides gras à chaîne carbonée comprise entre C12 et C24. L'étape finale est le clivage par un acide fort, tel que l'acide fluorhydrique anhydre, des conjugués synthétisés sur support solide, tel qu'une résine.

Cette méthode de synthèse améliorée permet une production plus efficace de certains acides aminés en évitant leur implication dans des réactions de terminaison précoce.

L'association desdits peptides aux acides gras peut être réalisée soit en position N-terminale ou alternativement soit en position C-terminale.

Avantageusement, dans les conjugués, l'association des peptides aux acides gras est réalisée en position N-terminale et C-terminale des séquences peptidiques, en particulier pour donner des séquences tétrapalmitoylées.

Alternativement ou cumulativement, on peut envisager d'associer des acides gras à des résidus internes dans la séquence peptidique.

Chaque peptide de la composition immunogène est associé à plusieurs molécules d'acide gras par le biais d'une ou de plusieurs molécules de PEG.

Les conjugués obtenus sont ensuite présentés à la surface des liposomes.

Optionnellement, les conjugués une fois synthétisés sont purifiés, par exemple par RP-HPLC ou chromatographie liquide à haute performance à polarité de phase inversée. Cette étape de purification est rendue délicate en raison de la présence de chaîne lipidique qui entraine un élargissement des pics chromatographiques et des problèmes de solubilité. L'isolement du produit attendu, des impuretés formées lors de l'élongation du peptide peut être délicat et conduire à des rendements faibles. En outre, la purification peut être difficile dans la mesure où l'acide gras est situé en position C-terminale. En effet, dans ce cas le produit désiré mais aussi les impuretés portent la partie lipophile à l'origine des difficultés chromatographiques. De plus, comme cette stratégie fait Intervenir en fin de synthèse une étape dé coupure et de déprotection en milieu fort, ce traitement limite le choix de la partie lipophile (Deprez et coll. 1996. Vaccine 14(5) : 375-382 ; Stöber et coll. (1997) Bioorg. Med. Chem. 5(1): 75-83).

La présentation à la surface des liposomes des conjugués obtenus est ensuite obtenue de manière mécanique. En effet, les conjugués mélangés aux liposomes s'enchassent au sein de la membrane phospholipidique des liposomes à l'aide de leurs doubles chaînes lipidiques.

La présente invention concerne également la mise en oeuvre de cette composition dans des méthodes d'immunisation selon l'invention.

La présente invention porte donc sur une méthode d'immunisation comprenant une première administration, notamment par injection de la composition immunogène selon l'invention, et une administration de rappel de ladite composition (ou booster) par exemple de deux injections successives. Les injections de rappel, exposant plusieurs fois le même antigène, induisent une réponse immunitaire secondaire forte. L'exposition répétée de peptides dérivés des boucles extracellulaires de la protéine P-170 auprès du système immunitaire induit une mémoire immunologique ainsi que des réponses secondaires ultérieures rapides et élevées en titre d'anticorps.

Plus particulièrement, dans la présente méthode d'immunisation les injections chez l'humain sont réalisées à 1 mois d'intervalle.

Selon un mode de réalisation, l'immunisation par la composition selon l'invention peut être mise en oeuvre de manière concomitante ou précédent un traitement anticancéreux administré à un patient.

De préférence, l'immunisation précède le traitement de chimiothérapie afin de prévenir et anticiper l'apparition d'un phénotype de résistance multidrogues chez le patient. Ce plan de traitement sera préféré à une immunisation concomitante aux traitements chimiothérapeutiques lorsque le diagnostic et l'évolution du cancer permettent une prise en charge thérapeutique curative (chimiothérapie) décalée d'au moins 30 jours à compter de la date de diagnostic. Une prise en charge tardive du cancer n'empêche pas l'utilisation due la méthode d'immunisation par la composition de la présente invention. En effet, l'association dans le même temps du traitement anticancéreux et de l'immunisation par ladite composition immunogène induit néanmoins une réponse immunitaire pour la production d'auto-anticorps anti-P-170 ayant un effet curatif ou palliatif sur l'apparition du phénotype de résistance multidrogues. L'effet curatif de l'immunisation par la présente composition s'illustre par une réversion du phénotype de résistance multidrogues. Dans le contexte de l'invention, l'expression « réversion » du phénotype de résistance multidrogues désigne le passage d'un phénotype de résistance multidrogues à un phénotype dit sensible aux traitements chimiothérapeutiques.

Par « chimiothérapie », « anticancéreux » ou « drogues chimiothérapeutiques », l'on entend au sens de la présente invention, tout traitement curatif ou palliatif de tumeurs primaires ou secondaires à base d'agents cytotoxiques. La chimiothérapie requiert généralement plusieurs cycles de traitement.

Dans le cadre d'une méthode d'immunisation précédent le traitement de chimiothérapie, la mise en oeuvre de la première injection de la composition selon la présente invention précède d'au moins 60 jours, de préférence 63 et 67 jours, le début du traitement chimiothérapeutique.

Les compositions selon l'invention sont administrables par voie topique, par voie systémique (orale, nasale et autres voies muqueuses) et/ou par voie parentérale (intraveineuse, sous-cutanée, intramusculaire ou intra-péritonéale) ou par combinaison de ces voies et induisent effectivement un réponse immune protectrice contre la résistance multidrogues. La composition est formulée de manière à permettre une administration aisée par les diverses voies ci-dessus. En particulier, le choix des composés auxiliaires (agent mouillant, émulsifiant ou tampon) est dicté par le mode d'administration choisi.

Avantageusement, l'immunisation est réalisée grâce à une administration par voie intramusculaire et intra-péritonéale respectivement chez l'homme et chez la souris.

Un des objets de la présente invention concerne également la mise à disposition d'un anticorps, en particulier un auto-anticorps, induit contre la P-170 humaine ou murine, qui se lie de façon spécifique aux peptides des conjugués selon l'invention.

Cet anticorps peut être un anticorps polyclonal ou monoclonal, sélectionné parmi le groupe comprenant les isotypes IgG1, IgG2, IgG3 et un IgM. En particulier l'anticorps IgG2 peut être du sous type 2a ou 2b.

La présente invention porte en outre sur une composition immunogène comprenant d'une part un véhicule et d'autre part des conjugués comprenant au moins un peptide dérivé de la boucle extracellulaire 1 de la protéine P-170 permettant l'induction d'anticorps anti-mpp1, chaque peptide étant associé à plusieurs molécules d'acide gras de chaîne carbonée comprise entre C12 et C24 lesdits conjugués présentant tout ou partie de la conformation de la boucle extracellulaires 1 de la protéine P-170, pour la réversion de la résistance multidrogues apparaissant chez un patient atteint d'un cancer.

Cette composition est particulièrement adaptée aux traitements des tumeurs solides exprimant le gène MDR11 codant pour la protéine P-170 humaine.

La présente invention concerne également l'utilisation de la composition immunogène selon l'Invention pour la fabrication d'un vaccin destiné au traitement et/ou la prévention d'une résistance multidrogues apparaissant chez un patient atteint d'un cancer. Le cancer tel qu'envisagé sera un cancer du rein, du foie, du colon, de l'intestin, de la prostate, du sein, de la vessie, du cerveau, du sang (leucémie) et/ou des tissus médullaires (myélome). Il pourra également être une tumeur solide exprimant le gène MDR1 codant pour la protéine P-170 humaine. L'utilisation de la composition immunogène pourra aussi se faire en association avec un traitement anticancéreux.

En particulier, il est prévu lors des phases cliniques I/II d'utiliser le présent vaccin chez des patients porteurs de tumeurs solides, en particulier les cancers exprimant le gène MDR1 codant pour la protéine P-170 humaine, de type cancer du rein, du foie, du colon, de l'intestin, de la prostate, du sein, de la vessie, du cerveau, du sang (leucémie) et/ou des tissus médulaires (myélome). Les patients choisis pour cette étude clinique seront sélectionnés sur des critères dé non-immunodépression et de tolérance à un traitement standard. Parallèlement à ces essais, sera effectuée une étude pharmacodynamique et de tolérance chez ces mêmes patients.

Enfin, la composition selon la présente invention sera utilisée directement chez des patients exprimant spontanément une résistance multidrogues afin d'évaluer cliniquement chez l'homme le taux de réversion du phénotype de résistance muludrogues.

La présente invention comprend également une méthode de traitement et/ou de prévention d'une résistance multidrogues apparaissant chez un patient atteint d'un cancer, en particulier un cancer atteignant le rein, le foie, le colon, l'intestin, la prostate, le sein, la vessie, le cerveau; le sang (leucémie) et/ou les tissus médulaires (myélome) comprenant l'administration de la composition immunogène telle que décrite.

Le cancer particulièrement visé par le traitement tel que décrit est une tumeur solide exprimant le gène MDR1 codant pour la protéine P-170 humaine.

Finalement, un kit d'exploitation de la composition immunogène, comprenant la composition immunogène selon l'invention et, optionnellement, des réactifs et/ou des instructions d'utilisation est également envisagé.

L'un quelconque des aspects ou modes de réalisation décrit précédemment qui dépassent la portée de l'invention représenté par les revendications ne fait pas partie de l'invention mais présente le contexte utile pour comprendre l'invention.

### Exemples

### I-1 Préparation des conjugués formés Par liaison covalente entre la région peptidique et les molécules d'acide gras de chaîne carbonée comprise entre C12 et C24.

La synthèse des peptides peut être réalisée sur un synthétiseur de peptides, par exemple un synthétiseur « Applied Biosystems 430A » en utilisant le (13,14) tertiobutyloxycarbonyl/benzyl est *in situ* une activation par le (N-[(1H-benzotriazol-1-yl)(diméthylamino)méthylène]-N- méthylméthanaminium hexafluorophosphate N-oxyde (Schölzer M. et al Science 1992, 256 (5054) : 221-225), puis couplés de façon covalente avec quatre molécules d'acides palmitique par molécule de peptide (figure 1).

Une méthode de synthèse améliorée permettant une production plus efficace de certains acides aminés en évitant leur implication dans des réactions de terminaison précoce a été développée. Ceci est particulièrement intéressant dans le cas de mpp1 du fait de sa longueur (43 acides aminés). Les peptides murins mpp1, mpp2 et mpp4 ont notamment été synthétisés et les résultats de la synthèse (analyse de la séquence des peptides, masse moléculaire, et pureté) ont été rapportés au Tableau 1 *supra,* chaque peptide ayant été contrôlé pour sa séquence par analyse des acides aminés après hydrolyse acide totale, pour sa masse moléculaire par analyse de spectrométrie de masse et pour sa pureté par HPLC.

Selon cette méthode, la synthèse des conjugués repose sur l'élaboration sur résine de la séquence peptidique voulue, puis la déprotection des fonctions amines des lysines N et C-terminales afin de les coupler avec les acides gras à chaîne carbonée comprise entre C12 et C24. L'étape finale est le clivage par l'acide fluorhydrique anhydre. Les conjugués ont été obtenus en utilisant la stratégie Boc/benzyle. L'approche optimale consiste à élaborer la séquence peptidique sur la phase solide puis à coupler un acide gras activé sur une fonction amine (ou thiol) déprotégée sélectivement. Si l'acide gras est introduit à l'extrémité N-terminale, le peptide ne nécessite pas d'aménagements fonctionnels particuliers. Par contre l'introduction de l'acide gras en position C-terminale s'effectue en général sur la fonction amino ε d'une chaine latérale de lysine. En stratégie Boc/benzyle, il est nécessaire d'introduire l'acide aminé Boc-L-Lys(Fmoc)-OH lors de la synthèse du peptide. Après avoir élaboré toute la séquence, la fonction amino est déprotégée puis acylée avec l'acide gras de chaîne carbonée comprise entre C12 et C24. Le conjugué est enfin déprotégé et coupé de la résine en présence d'un acide fort, l'acide fluorhydrique anhydrique (Figure 2).

### I-2 Evaluation du degré d'immunogénicité des peptides couplés à deux ou quatre acides gras de chaîne carbonée comprise entre C12 et C24

Afin de progresser dans la compréhension du degré d'immunogénicité des conjugués salon l'invention, deux types de conjugués ont été fabriqués et testés. Le premier type de conjugués correspond aux séquences synthétiques des peptides dérivés des boucles extracellulaires de la protéine P-170 murine, couplées de façon covalente à quatre molécules d'acide gras, par molécule de peptide, de chaîne carbonée comprise entre C12 et C24. Le second type de conjugués est formé d'un peptide couplé seulement à deux molécules d'acide gras. Cette étude est illustrée à l'aide d'un exemple spécifique de conjugués dipalmitoylés et tétrapalmitoylés.

Le tableau III décrit les séquences des conjugués di- et tétrapalmitoylés correspondant aux boucles 1, 2 et 4 de la protéine P-170 murine ainsi que leur capacité immunogène mesurée à l'aide de la détection des anticorps par la technique de Dot Blot ainsi que le titre en anticorps par unités de fluorescence. On observe que les souris ayant reçu des liposomes contenant des conjugués avec deux résidus palmityls ne montrent pas de réponse immunitaire à l'exception du mpp'4, correspondant à la boucle 4 de la protéine P-170 murine. Le titre en anticorps induit par ce conjugué est toutefois quatre fois inférieur au titre induit par mpp4, soit la même séquence couplée à quatre résidus palmityls. Les conjugués mpp4 et mpp2 engendrent une réponse immunitaire avec un titre en anticorps proche de 400 unités de fluorescence. Dans le cadre de la première synthèse peptidique aucun anticorps n'a été détecté suite à l'injection du conjugué mpp1 correspondant à la séquence acide aminé la plus longue, ce qui pouvait résulter de la synthèse peptidique. En effet, la synthèse de cette boucle est très dure et longue puisqu'il existe de nombreuses terminaisons chimiques possibles,ou de repontage chimique ce qui pourrait expliquer le manque de réponse immunitaire de ce premier lot de peptides. Pour les essais de vaccination des animaux, il a donc été procédé à une nouvelle synthèse des peptides, notamment de la boucle 1.

Au vu de ces résultats, le modèle couplant chaque molécule de peptides à quatre molécules d'acide gras de chaîne carbonée comprise entre C12 et C24 renforce la capacité immunogène desdits conjugués incorporés dans la membrane des liposomes dans la composition immunogène selon l'invention (Figure 1). La structure tertiaire induite par les interactions hydrophobes aux extrémités N et C terminales joue donc un rôle pour l'induction d'une réponse immunitaire humorale importante et spécifique. Ces interactions de type hydrophobe sont assez fortes pour créer une conformation en boucle définie comme étant la structure tertiaire équivalente de la structure naturelle des boucles extracellulaires de la protéine P-170. Cette conformation « naturelle » se retrouve exposée de façon stable à la surface du véhicule, de préférence à la surface des liposomes, dans le cas des conjugués couplés à quatre résidus d'acide gras en C12 à C24 par molécule de peptides. La diminution par un facteur deux de ces interactions hydrophobes peut être insuffisante pour obtenir la structure « naturelle » conduisant à une inhibition presque totale du potentiel antigénique des conjugués selon l'invention.

**Tableau III :**

| Nom des conjugués | Séquences en acides aminés | Nombre de chaînes palmityles | Détection des anticorps par Dot Blot | Titre en Ac unités de fluorescence |
|---|---|---|---|---|
| mpp1 | | 4 | - | <10 |
| mpp'1a | G-GNMTDSFTKAEAS-G-K-NH2 | 2 | pas testé | pas testé |
| mpp'1b | G-LPSITNQSGPNS-G-K-NH2 | 2 | - | <10 |
| mpp'1c | G-TLIISNSSLEEE-G-K-NH2 | 2 | - | <10 |
| mpp'2 | | 2 | - | <10 |
| mpp2 | | 4 | + | 360 |
| mpp'4 | | 2 | + | 100 |
| mpp4 | | 4 | + | 400 |

### I-3 Préparations vaccinales

Les compositions immunogènes préparées comprennent :
Lp1 : conjugués, liposomes (DPMC, DPMG, cholestérol).
Lp2 : liposomes (DPMC, DPMG, cholestérol)
Lp3 : liposomes (DPMC, DPMG, cholestérol), MPLA, conjugués
Lp4 : conjugués.

Les liposomes présentent à leur surface les trois conjugués mpp1, mpp2 et mpp3 ajoutés dans un rapport molaire de 1 :250 avec les phospholipidés. Les solvants organiques permettant l'homogénéisation de cet ensemble sont évaporés, le film résultant, après hydratation avec du PBS pH=7,4 stérile est ajusté à une concentration finale en phospholipides de 4mM Enfin, les liposomes en suspension sont au moment de l'immunisation mélangés à de l'alum stérile (Pasteur Mérieux) dans un rapport volumique. Les préparations vaccinales injectées aux animaux correspondent donc aux compositions immunogènes Lp1, Lp2, Lp3 et Lp4 dans des formulations comprenant l'alum comme adjuvant de l'immunité, l'alum étant en outre capable de prolonger la durée d'absorption du vaccin.

### I-4 Animaux

Les études ont été réalisées sur des souris B6D2F1 femelles âgées de 6 à 10 semaines et issues de croisements entre des femelles C57B1/6 et des males DBA/2 (Charles River Laboratories). Les souris, objet de l'expérimentation, pèsent entre 19 et 22g. Les prélèvements sanguins chez l'animal sont réalisés, 7 à 12 jours après l'immunisation, au niveau du sinus rétro-orbital.

### I-5 Protocole d'immunisation

Les souris ont été immunisées trois fois à deux semaines d'intervalle par injection intra-péritonéale, avec 200µl de composition vaccinale. Ce protocole expérimental est conduit pour les quatre préparations sur des groupes de neuf souris B6D2F1 (Iffa Credo L'Arbresle, France).

Pour quantifier par Dot Blot les différentes immunoglobulines induites par la vaccination, on a prélevé pour chaque souris, 100µl de sang au sinus rétro-orbital, un jour avant le rappel et 15 jours après la dernière injection. Chaque échantillon de sang a ensuite été centrifugé et le sérum isolé a été utilisé pour la quantification.

### I-6 Agents anticancéreux

La doxorubicine (Dox) (Sigma) et la vinblastine (VLB) sont utilisées en tant qu'agents anticancéreux dans le protocole de modèle *in vivo* d'induction de tumeurs solides et de chimiothérapie après immunisation.

La doxorubicine est le chef de file des antinéoplasiques cytostatiques de la famille des anthracyclines, elle est donc largement utilisée seule ou en association dans le traitement de nombreuses tumeurs. Le mode d'action principal de la doxorubicine semble être au niveau de l'inhibition de l'ADN topoisomérase II. Mais comme tous les médicaments anticancéreux, la doxorubicine présente des effets secondaires en particulier d'ordre hématologique, digestif, inflammatoire et surtout toxicité cardiaque qui en limite l'usage dans les traitements chimiothérapeutiques. La solution de doxorubicine est utilisée à une concentration de 10⁻³mol/l dans les présentes expérimentations.

La vinblastine est un vinca alcaloïde couramment utilisé en thérapeutique en tant qu'agent bloquant les mitoses cellulaires en métaphase, d'où son nom de poison du fuseau mitotique. La vinblastine tue donc préférentiellement les cellules qui se divisent rapidement, elle est donc particulièrement appropriée pour le traitement du cancer des testicules et le sarcome de Kaposi. Néanmoins, les manifestations toxiques de la vinblastine sont nombreuse et variées, la principale de ces manifestations étant la toxicité sanguine. Enfin, la solution de vinblastine est utilisée à une concentration de 10⁻² mol/l dans les essais expérimentaux de la présente invention.

### I-7 Modèle in vivo d'induction de tumeurs solides

La lignée cellulaire B16R, originaire d'un mélanome murin et sélectionnée pour sa résistance à la doxorubicine, a été choisie dans la présente invention pour le développement de tumeurs solides. Les cellules B16R sont cultivées *in vitro,* récoltées en phase exponentielle de croissance et nettoyées avec un tampon salin phosphaté (PBS) avant leur administration par injection sous cutanée dans le flanc arrière de la souris. Le volume d'injection est de 50µl d'une suspension de 1.10⁶cellules B16R en NaCl 0.85% (Candido KA et al Cancer Res 2001, 61 (1) :228-236). Les souris développent un mélanome de taille moyenne de 2.0g ± 1.2g dans une période comprise entre 22 et 24 jours après inoculation de cellules cancéreuses.

Après développement tumoral, la lignée B16R s'est confirmée résistante aux traitements chimiothérapeutiques avec la doxorubicine. Dès lors les conditions expérimentales précédentes ont servi de modèle pour induire une tumeur solide à partir de cellules P388R murines (cellules de néoplasme lymphoïde murin caractérisées et utilisées comme cellules de référence pour leurs propriétés MDR - Kohls WD. et al Cancer Res 1986 Sep, 46(9) :4352-6) résistantes également à la doxorubicine .

D'autres cellules tumorales peuvent être mises en oeuvre dès lors qu'elles présentent une résistance à la chimiothérapie testée.

### I-8 Protocole de chimiothérapie après immunisation

Un protocole de chimiothérapie utilisant deux agents anticancéreux, la vinblastine et la doxorubicine, a été établi tel que décrit Figure 3. Le traitement chimiothérapeutique des souris préalablement immunisées avec les préparations vaccinales Lp1 et Lp2 débute un jour après l'injection sous-cutanée de 10⁶ cellules cancéreuses P388R (jour 0), par une injection hebdomadaire de doxorubicine à la dose de 5,5 mg/kg (jours 1, 10 et 22) suivi de l'injection alternée de vinblastine à la dose de 2,5mg/kg (jours 4 et 14). Pendant cette période la prise de nourriture, d'eau et le poids des souris ainsi que leur survie ont été enregistrés. Avant l'injection avec les cellules P388R, des échantillons de sérum de souris ont été prélevés pendant une période comprise entre 15 et 45 jours après l'immunisation, pour quantifier les anticorps anti-P170 et contrôler leur activité.

### I-9 Analyse de la réponse immunitaire par Dot-Blot

Les conjugués selon l'invention servant de molécules antigéniques, dilués dans du PBS, sont dans un premier temps déposés à température ambiante sur les membranes de nitrocellulose. Au bout de 30 minutes, ces molécules antigéniques sont bloquées avec 3ml d'une solution contenant du PBS-5% lait écrémé. Les membranes sont incubées pendant 2 heures, à température ambiante, sans lavage, avec 24µl de sérum murin dilué préalablement volume à volume avec du PBS, dans 2ml de PBS contenant 1% de lait écrémé et 0,1% de tween 20. Ledit sérum murin a été prélevé dans une période de 15 à 45 jours après la troisième immunisation. Après trois lavages en PBS-1 % lait écrémé-0.1 % tween 20, les membranes sont séquentiellement incubées pendant 1 heure à température ambiante, dans 3ml de PBS-1% lait écrémé-0.1% tween 20 contenant l'anticorps anti-souris péroxydase secondaire dilué au 1/3000^{ème}, puis après 2 lavages, dans 3ml de PBS-1% lait écrémé-0.1% tween 20. Les membranes sont ensuite lavées une fois 10 minutes dans du PBS seul et gardées une nuit au réfrigérateur à 4°C dans 500µl de PBS. Un substrat pour la péroxydase chimioluminescence (kit ECL^{™}, AMERSHAM Pharmacie Biotech) est déposé à la surface des membranes (0.125ml/cm²), et laissé pendant une minute, puis les membranes sont égouttées et placées en cassette « froide » entre 2 films Saran®. Les membranes sont immédiatement exposées en autoradiographie, la lumière émise résultant de la réaction d'oxydation du luminol par la peroxydase étant collectée sur des films KODAK X-OMAT avec des temps variants de quelques minutes a 1 heure selon l'importance du signal. Les titres en anticorps sont estimés à l'aide d'un densitomètre adapté au système supra. La sensibilité de la réaction de chimioluminescence présente un seuil de détection des anticorps induits évaluée à 0,2ng/ml dans ces conditions expérimentales.

Les protocoles des expérimentations pour évaluer la réponse immunitaire *in vivo* chez les souris immunisées par les préparations vaccinales décrites supra utilisent des anticorps anti-mpp1, mpp2, mpp4 ainsi que des anticorps secondaires anti-murin apécifiques Ig (M, G3, G2a, G2b, G1).

### I-10 Réponse immunitaire in vivo chez les souris B6D2F1 immunisées par les préparations vaccinales

La réponse immunitaire des souris B6D2F1 immunisées par le vaccin contrôlé Lp2 est prédominante en anticorps IgM. La concentration des anticorps IgM reste constante au cours des trois immunisations témoignant d'une réponse immunitaire de type aspécifique polyclonale due à la présence de MPLA. La valeur des anticorps IgM à été soustraite à celle trouvée dans les sérums de souris immunisées par le vaccin Lp1 (Figure 4).

Les souris immunisées par la préparation vaccinale Lp4 présentent des anticorps IgM anti-mpp1, anti-mpp2 et anti-mpp3 suite à la première immunisation. Cette quantité d'anticorps IgM diminue jusqu'à disparaître suite à la troisième immunisation pour laisser apparaître une réponse immunitaire prépondérante en anticorps IgG1 (Figure 5).

L'immunisation des souris par le vaccin Lp3 induit l'expression d'anticorps IgM. après la première injection. Le titre en anticorps IgM diminue après la deuxième immunisation, période pendant laquelle la réponse immunitaire devient prépondérante en anticorps IgG2b. Après la troisième injection le titre en anticorps IgG1 est maximal, en outre cette réponse immunitaire est positive pour les trois conjugués, le conjugué mpp2 étant le plus immunogène (Figure 6).

L'immunisation des souris par la préparation vaccinale Lp1 induit l'apparition prédominante d'anticorps IgM dirigé contre les boucles extracellulaires 1, 2 et 4 de la protéine P-170. La quantité d'anticorps IgM diminue au cours des deuxième et troisième immunisations pour laisser apparaître une réponse immunitaire prépondérante en anticorps IgG anti-mpp2. Les titres en anticorps IgG3, IgG2a et IgG2b sont environ deux à trois fois supérieurs à la valeur basale alors que le titre en anticorps IgG1 est cinq fois plus important (Figure 7).

On observe, par comparaison des quantités d'anticorps IgG1, que le conjugué mpp2 est respectivement 2.6 et 2 fois plus immunogène que les conjugués mpp1 et mpp4. De plus, on constate que la préparation vaccinale Lp1 induit la plus forte réponse immunitaire globale, soit les isotypes (IgM, G3, G2a, G2b, G1) correspondant à chacune des boucles extracellulaires 1,2 et 4 confondues.

Les titres en anticorps induits par le conjugué mpp1 sont significatifs et comparables en valeur aux titres en anticorps détectés pour les conjugués mpp2 et mpp4 contrairement aux résultats observés avec Tosi et coll. (1995. Biochemical and biophysical research communications 212(2) : 494-500).

Après avoir déterminé la préparation vaccinale Lp1 comme ayant le meilleur pouvoir immunogène, son innocuité a été contrôlée sur une période de 18 mois après la dernière immunisation. Les souris immunisées ne présentent pas de variations de poids significatives par rapport aux souris immunisées par le vaccin contrôle Lp2. De plus, aucune modification comportementale, par exemple au égard de la vigilance ou de l'appétit n'a été observée chez les animaux vaccinés avec la préparation vaccinale Lp1. Enfin, des analyses histopathologiques des organes exprimant de façon naturelle la protéine P-170 (rate, foie, reins, glandes surrénales, pancréas, ovaires, coeur et poumons) ont démontré l'absence de toxicité induite et/ou d'auto-immunité chez les souris immunisées par la préparation vaccinale Lp1. En effet, les seules lésions observables en position intra péritonéales et en périphérie des organes (foie, pancréas, rate et ovaires) ont été attribuées exclusivement à l'utilisation d'alum dans la composition immunogène. Des analyses complémentaires sur la recherche de l'agent d'induction des lésions observées ont confirmé ce résultat.

### I-11 Evaluation in vivo de l'activité anti-chimiorésistance associée à l'immunisation par la préparation vaccinale Lp1

L'étude *in vivo* de l'évolution du phénotype de résistance multidrogues chez les souris immunisées par les préparations vaccinales Lp1 et Lp2 (contrôlé) a été initiée suivant un protocole d'induction de tumeurs solides suivi du plan de traitement chimiothérapeutique. Le traitement anticancéreux débute 1 jour après l'inoculation des cellules cancéreuses.

Préalablement à l'injection des cellules P 388R aux souris immunisées, les titres en anticorps dans les sérums des souris immunisées avec la préparation vaccinale Lp1 ont été déterminés : respectivement 100%, 40% et 80% des sérums présentaient des anticorps de type IgG1 anti-mpp1,2 et 4 avec une valeur moyenne de 0,3, 0,21 et 0.33 µg/ml (1U correspond à 0.2µg/ml).

Le temps de survie des souris immunisées par le vaccin Lp1 et Lp2 a été représenté en fonction du temps (Figure 8),

Les résultats graphiques montrent que le temps moyen de survie du groupe de souris immunisées par Lp1 est de 39 jours alors que dans le groupe vacciné par la préparation Lp2, il est de 22 jours. Les souris immunisées par la préparation vaccinale Lp1 de façon préventive ont donc une augmentation de 77% du temps moyen de survie par rapport au contrôle.

Dans le groupe Lp2, on a observé pour l'une des souris, un temps de survie de 70 jours.

Cette augmentation de 77% du temps de survie est observée alors même que le traitement chimiothérapeutique en tant que tel n'a été administré que 22 jours à compter de l'injection des cellules cancéreuses résistantes. Or, on observe que le taux de survie chute à compter de la fin de l'administration des anticancéreux, en conséquence on peut en déduire qu'une réversion complète serait observée si le traitement chimiothérapeutique se poursuivait sachant que seul ce dernier a un effet curatif contrairement aux auto-anticorps obtenus chez le patient immunisé par la composition selon la présente invention.

Ces résultats sont très prometteurs puisque les meilleurs résultats publiés obtenus dans le traitement de la résistance multidrogues avec le même modèle de cancer décrivaient une augmentation de survie de 49% chez des souris traitées par le S9788 aux doses de 100mg/kg/jour (Pierré et coll. 1992. Invest New Drug. 10 : 137-148). De plus, Yang et coll. (1999. BBRC. 266 : 167-173) ont observé, avec la même lignée cellulaire, une augmentation de survie de 35% chez des souris traitées par la vincristine et la ciclosporine A. D'autres auteurs ont encore démontré que certains révertants comme le trans-flupenthixol peuvent accélérer la mortalité par une augmentation du potentiel invasif des cellules cancéreuses.

Cette augmentation du temps de survie est d'autant plus appréciable que les modèles expérimentaux murins de cancers sont très exigeants sur l'efficacité du traitement puisque dès l'inoculation les cellules cancéreuses ont un degré de résistance supérieur à celui observé en clinique. Cette constatation implique que les agents de réversion soient actifs dès l'injection des cellules cancéreuses, or on note que ces agents sont généralement actifs à des concentrations cytotoxiques atteintes progressivement au cours du traitement.

L'immunisation par la préparation vaccinale Lp1 induit chez les souris la formation d'auto-anticorps actifs capables d'inhiber *in vivo* rapidement et durablement la résistance à la chimiothérapie. L'immunisation par le vaccin Lp1 permet donc d'inhiber rapidement la chimiorésistance et de rétablir *in vivo* l'activité des anticancéreux chez les patients devenus réfractaires au traitement chimiothérapeutique. Au cours d'expérimentations complémentaires, les auto-anticorps circulants chez les souris immunisées par la préparation Lp1 n'ont induit, aucune cytotoxicité, aucun développement de lésions auto-immunes ni de majoration du potentiel invasif des cellules cancéreuses.

Les descriptions qui précèdent mettent en particulier en oeuvre des peptides de la protéine P-170 murine. Les protocoles décrits sont cependant naturellement applicables de façon similaire à la synthèse de tout autre peptide, notamment ceux qui ont été décrits ci-dessus pour la protéine P-170 humaine.

## Revendications

1. Composition immunogène comprenant un véhicule et , en tant que structure antigénique, des conjugués comprenant tout ou partie des séquences d'acides aminés d'au moins un peptide dérivé d'une boucle extracellulaire de la protéine P-170, chaque peptide étant associé à au moins deux molécules d'acide gras de chaîne carbonée comprise entre C12 et C24, **caractérisée en ce que** le ou lesdits peptides sont associés aux molécules d'acides gras par le biais d'une ou de plusieurs molécules de polyéthylène glycol (PEG), liées aux résidus lysine (K) se trouvant en positions N- et C-terminales des séquences d'acides aminés d'au moins un peptide dérivé d'une boucle extracellulaire de la protéine P-170, dès lors que les séquences d'acides aminés permettent, dans des conditions d'administration appropriées, l'induction d'anticorps anti P-170.

2. Composition immunogène selon la revendication 1, **caractérisée en ce qu'**elle permet l'induction d'anticorps anti-P-170 pour la réversion de la résistance multidrogues apparaissant chez un patient atteint d'un cancer.

3. Composition immunogène selon les revendications 1 et 2, **caractérisée en ce que** les conjugués comprennent tout ou partie des séquences d'acides aminés d'au moins deux boucles extracellulaires de la protéine P-170.

4. Composition immunogène selon les revendications 1 et 2, **caractérisée en ce que** les conjugués comprennent tout ou partie des séquences d'acides aminés d'au moins trois boucles extracellulaires de la protéine P-170.

5. Composition immunogène selon les revendications 1 à 4, dans laquelle les conjugués comprennent tout ou partie des séquences d'acides aminés sélectionnés parmi les peptides dérivés des boucles extracellulaires 1, 4 et 6 de la protéine P-170 humaine, lesdits conjugués permettant l'induction d'anticorps anti-P-170 humains (anti-hpp).

6. Composition immunogène selon la revendication 5, dans laquelle les conjugués comprennent tout ou partie des séquences d'acides aminés de peptides dérivés de la boucle 1 de la protéine P-170 humaine correspondant aux trois peptides, 1 a, 1b et 1c, résultant de la coupure de ladite boucle 1 extracellulaire au niveau des sites de glycosylation.

7. Composition immunogène selon les revendications 1 à 4, dans laquelle les conjugués comprennent tout ou partie des séquences d'acides aminés sélectionnés parmi les peptides dérivés des boucles extracellulaires 1, 2 et 4 de la protéine P-170 murine, lesdits conjugués permettant l'induction d'anticorps anti-P-170 murins (anti-mpp).

8. Composition immunogène selon les revendications 1 à 6, **caractérisée en ce que** tout ou partie des séquences d'acides aminés des peptides des conjugués sont respectivement choisis parmi les séquences d'acides aminés suivantes :
pour la boucle 1 :
le peptide 1 SEQ ID NO 4:
GEMTDIFANAGNLEDLLMSNITNRSDINDTGFFMNLEEDMTRYAYYYS
ou le peptide 1a SEQ ID NO 5: GEMTDIFANAGNLEDLLMS.
ou le peptide 1b SEQ ID NO 6: NITNRSDINDTGFF
ou le peptide 1c SEQ ID NO 7: MNLEEDMTRYAYYYS
pour la boucle 4 : SEQ ID NO 8: FSRIIGVFTFTRIDDPETKRQNSNLFS
pour la boucles 6 : SEQ ID NO 10: FRFGAYLVAHKLMSFED
et/ou leur combinaison, et/ou les séquences d'acides aminés modifiées, dès lors que les séquences d'acides aminés ainsi modifiées permettent, dans des conditions d'administration appropriées, l'induction d'anticorps anti-P-170.

9. Composition immunogène selon les revendications 1 à 8, **caractérisée en ce que** une molécule de PEG, de 1 à 8000 monomètres, est utilisée.

10. Composition immunogène selon les revendication 1 à 6, **caractérisée en ce que** tout ou partie des séquences d'acides aminés des peptides des conjugués sont respectivement choisis parmi les séquences d'acides aminés suivantes :
pour la boucle 1 :
le peptide 1 SEQ ID NO 1:
GNMTDSFTKAEASILPSITNQSGPNSTLIISNSSLEEE
pour la boucle 2 SEQ ID NO 2: KVLTSFTNKELAYAK
pour la boucle 4 : SEQ ID NO:3: SRDDDMETKRQNEN
et/ou leur combinaison, et/ou les séquences d'acides aminés modifiées, dès lors que les séquences d'acides aminés ainsi modifiées permettent, dans des conditions d'administration appropriées, l'induction d'anticorps anti-P-170.

11. Composition immunogène selon les revendication 1 à 10, **caractérisée en ce que** chaque peptide étant lié de manière covalente à au moins deux chaînes de polyéthylène glycol.

12. Composition immunogène selon la revendication 11, **caractérisée en ce que** les deux chaînes de polyéthylène glycol sont couplées chacune a une molécule de phosphatidyl éthanolamine.

13. Composition immunogène selon les revendications 1 à 12, **caractérisée en ce que** le véhicule choisi pour présenter les conjugués est choisi dans le groupe consistant en des liposomes, des protéines membranaires bactériennes, des protéines Omp d'entérobactéries, des nanoparticules, des miscelles, des particules d'or, des microbilles et des virosomes

14. Composition immunogène selon la revendication 13, **caractérisée en ce que** le véhicule choisi consiste en des liposomes.

15. Composition immunogène selon la revendication 14, dans laquelle les conjugués et les liposomes sont dans un rapport molaire compris entre 1/10 et 1/1000, de préférence de 1/250.

16. Composition immunogène selon les revendications 14 et 15, dans laquelle les liposomes sont de préférence obtenus par mélange des phospholipides dimyristoyle phosphatidylcholine (DPMC), glycérol de phosphatidyle de dimyristoyle (DPMG) et cholestérol.

17. Composition immunogène selon la revendication 16, dans laquelle le mélange de DPMC, DPMG est cholestérol est dans les proportions 0.9:0:1:0:7.

18. Composition immunogène selon l'une quelconque des revendications 1 à 17, comprenant en outre au moins un adjuvant.

19. Composition immunogène selon la revendication 18, dans laquelle l'adjuvant est choisi dans le groupe consistant en de l'alum, du phosphate de calcium, de l'interleukine 1, du monophosphoryl lipide A (MPLA) et/ou des microcapsules de protéines et de polysaccharides.

20. Composition immunogène selon la revendication 19, dans laquelle l'adjuvant est de préférence l'alum.

21. Composition immunogène selon l'une quelconque des revendications 1 à 20, pour l'utilisation dans le traitement et/ou la prévention d'une résistance multidrogues apparaissant chez un patient atteint d'un cancer.

22. Composition immunogène, pour l'utilisation selon la revendication 21, dans laquelle le cancer.atteint le rein, le foie, le colon, l'intestin, la prostate, le sein, la vessie, le cerveau, le sang (leucémie) et/ou les tissus médulaires (myélome).

23. Composition immunogène, pour l'utilisation selon la revendication 21. **caractérisée en ce que** le cancer est une tumeur solide exprimant le gène MDR1 codant pour la protéine P-170 humaine.

24. Composition immunogène selon l'une quelconque des revendications 1 à 20, pour l'utilisation dans le traitement d'une résistance multidrogues apparaissant chez un patient atteint d'un cancer traité au moyen de médicaments anticancéreux.

25. Composition immunogène pour l'utilisation selon l'une quelconque des revendications 21 à 24, **caractérisée en ce que** ledit traitement comprend une première administration, et une administration de rappel de ladite composition.

26. Composition immunogène pour l'utilisation selon la revendication 25, **caractérisée en ce que** la méthode d'immunisation est mise en oeuvre de manière concomitante ou précédent un traitement anticancéreux.

## Patentansprüche

1. Immunogene Zusammensetzung, die einen Träger und als Antigenstruktur Konjugate umfasst, die alle oder einen Teil der Aminosäuresequenzen von mindestens einem Peptid umfassen, das von einer extrazellulären Schleife des Proteins P-170 stammt, wobei jedes Peptid mit mindestens zwei Fettsäuremolekülen der Kohlenstoffkette zwischen C12 und C24 verbunden ist, die **dadurch gekennzeichnet ist, dass** das oder die Peptid(e) mit den Fettsäuremolekülen durch eines oder mehrere Polyethylenglycol (PEG)-Moleküle verbunden ist/sind, die an Lysinreste (K) gebunden sind, die sich an den N- und C-terminalen Positionen der Aminosäuresequenzen von mindestens einem Peptid befinden, das von einer extrazellulären Schleife des Proteins P-170 stammt, wenn die Aminosäuresequenzen unter geeigneten Verabreichungsbedingungen die Induktion von anti-P-170-Antikörpern ermöglichen.

2. Immunogene Zusammensetzung nach Anspruch 1, **dadurch gekennzeichnet, dass** sie die Induktion von anti-P-170-Antikörpern zur Umkehrung der Multidrug-Resistenz ermöglicht, die bei einem Individuum auftritt, das an Krebs leidet.

3. Immunogene Zusammensetzung nach den Ansprüchen 1 und 2, **dadurch gekennzeichnet, dass** die Konjugate alle oder einen Teil der Aminosäuresequenzen von mindestens zwei extrazellulären Schleifen des Proteins P-170 umfassen.

4. Immunogene Zusammensetzung nach den Ansprüchen 1 und 2, **dadurch gekennzeichnet, dass** die Konjugate alle oder einen Teil der Aminosäuresequenzen von mindestens drei extrazellulären Schleifen des Proteins P-170 umfassen.

5. Immunogene Zusammensetzung nach den Ansprüchen 1 bis 4, in der die Konjugate alle oder einen Teil der Aminosäuresequenzen umfassen, die aus den Peptiden ausgewählt sind, die von den extrazellulären Schleifen 1, 4 und 6 des humanen Proteins P-170 stammen, wobei die Konjugate die Induktion von humanen anti-P-170-Antikörpern (anti-hpp) ermöglichen.

6. Immunogene Zusammensetzung nach Anspruch 5, in der die Konjugate alle oder einen Teil der Aminosäuresequenzen von den Peptiden umfassen, die von der Schleife 1 des humanen Proteins P-170 stammen und die den drei Peptiden 1 a, 1b und 1 c entsprechen, die aus der Spaltung der extrazellulären Schleife 1 an den Glykosylierungsstellen resultieren.

7. Immunogene Zusammensetzung nach den Ansprüchen 1 bis 4, in der die Konjugate alle oder einen Teil der Aminosäuresequenzen umfassen, die aus den Peptiden ausgewählt sind, die von den extrazellulären Schleifen 1, 2 und 4 des murinen Proteins P-170 stammen, wobei die Konjugate die Induktion von murinen anti-P-170-Antikörpern (anti-mpp) ermöglichen.

8. Immunogene Zusammensetzung nach den Ansprüchen 1 bis 6, **dadurch gekennzeichnet, dass** alle oder ein Teil der Aminosäuresequenzen der Peptide der Konjugate jeweils aus den folgenden Aminosäuresequenzen ausgewählt sind:
für die Schleife 1:
das Peptid 1: SEQ ID NO:4:
GEMTDIFANAGNLEDLLMSNITNRSDINDTGFFMNLEEDMTRYAYYYS
oder das Peptid 1 a: SEQ ID NO:5: GEMTDIFANAGNLEDLLMS
oder das Peptid 1 b: SEQ ID NO:6: NITNRSDINDTGFF
oder das Peptid 1 c: SEQ ID NO:7: MNLEEDMTRYAYYYS
für die Schleife 4: SEQ ID NO:8: FSRIIGVFTRIDDPETKRQNSNLFS
für die Schleife 6: SEQ ID NO:10: FRFGAYLVAHKLMSFED
und/oder deren Kombination, und/oder den modifizierten Aminosäuresequenzen, wenn die so modifizierten Aminosäuresequenzen unter geeigneten Verabreichungsbedingungen die Induktion von anti-P-170-Antikörpern ermöglichen.

9. Immunogene Zusammensetzung nach den Ansprüchen 1 bis 8, **dadurch gekennzeichnet, dass** ein PEG-Molekül, das aus 1 bis 8000 Monomeren besteht, verwendet wird.

10. Immunogene Zusammensetzung nach den Ansprüchen 1 bis 6, **dadurch gekennzeichnet, dass** alle oder ein Teil der Aminosäuresequenzen der Peptide der Konjugate jeweils aus den folgenden Aminosäuresequenzen ausgewählt sind:
für die Schleife 1:
das Peptid 1: SEQ ID NO:1:
GNMTDSFTKAEASILPSITNQSGPNSTLIISNSSLEEE
für die Schleife 2: SEQ ID NO:2: KVLTSFTNKELAYAK
für die Schleife 4: SEQ ID NO:3: SRDDDMETKRQNEN
und/oder deren Kombination, und/oder den modifizierten Aminosäuresequenzen, wenn die so modifizierten Aminosäuresequenzen unter geeigneten Verabreichungsbedingungen die Induktion von anti-P-170-Antikörpern ermöglichen.

11. Immunogene Zusammensetzung nach den Ansprüchen 1 bis 10, **dadurch gekennzeichnet, dass** jedes Peptid an mindestens zwei der Polyethylenglycol-Ketten kovalent gebunden ist.

12. Immunogene Zusammensetzung nach Anspruch 11, **dadurch gekennzeichnet, dass** jede der zwei Polyethylenglycol-Ketten an ein Phosphatidylethanolamin-Molekül gebunden ist.

13. Immunogene Zusammensetzung nach den Ansprüchen 1 bis 12, **dadurch gekennzeichnet, dass** der Träger, der ausgewählt wurde, um die Konjugate zu präsentieren, ausgewählt ist aus der Gruppe bestehend aus Liposomen, bakteriellen Membranproteinen, OMP-Proteinen von Enterobakterien, Nanopartikeln, Mizellen, Goldpartikeln, Microbeads und Virosomen.

14. Immunogene Zusammensetzung nach Anspruch 13, **dadurch gekennzeichnet, dass** der ausgewählte Träger aus Liposomen besteht.

15. Immunogene Zusammensetzung nach Anspruch 14, in der die Konjugate und die Liposomen in einem Molverhältnis zwischen 1/10 und 1/1000, bevorzugt 1/250 sind.

16. Immunogene Zusammensetzung nach den Ansprüchen 14 und 15, in der die Liposomen bevorzugt durch Mischen der Phospholipide Dimyristoyl-Phosphatidylcholin (DPMC), Dimyristoylphosphatidylglycerin (DPMG) und Cholesterin erhalten werden.

17. Immunogene Zusammensetzung nach Anspruch 16, in der das Gemisch aus DPMC, DPMG und Cholesterin im Verhältnis von 0,9:0,1:0,7 vorliegt.

18. Immunogene Zusammensetzung nach einem der Ansprüche 1 bis 17, die des Weiteren mindestens ein Adjuvans umfasst.

19. Immunogene Zusammensetzung nach Anspruch 18, in der das Adjuvans ausgewählt ist aus der Gruppe bestehend aus Alum, Kalziumphosphat, Interleukin 1, Monophosphoryl-Lipid A (MPLA) und/oder Mikrokapseln von Proteinen und Polysacchariden.

20. Immunogene Zusammensetzung nach Anspruch 19, in der das Adjuvans bevorzugt Alum ist.

21. Immunogene Zusammensetzung nach einem der Ansprüche 1 bis 20 zur Verwendung bei der Behandlung und/oder Vorbeugung einer Multidrug-Resistenz, die bei einem Individuum auftritt, das an Krebs leidet.

22. Immunogene Zusammensetzung zur Verwendung nach Anspruch 21, in der der Krebs die Niere, die Leber, das Kolon, den Darm, die Prostata, die Brust, die Blase, das Gehirn, das Blut (Leukämie) und/oder Markgewebe (Myelom) betrifft.

23. Immunogene Zusammensetzung zur Verwendung nach Anspruch 21, **dadurch gekennzeichnet, dass** der Krebs ein solider Tumor ist, der das Gen MDR1 exprimiert, das das humane Protein P-170 codiert.

24. Immunogene Zusammensetzung nach einem der Ansprüche 1 bis 20 zur Verwendung bei der Behandlung einer Multidrug-Resistenz, die bei einem Individuum auftritt, das an einer Krebserkrankung leidet und das mit Anti-Krebs-Medikamenten behandelt wird.

25. Immunogene Zusammensetzung zur Verwendung nach einem der Ansprüche 21 bis 24, **dadurch gekennzeichnet, dass** die Behandlung eine primäre Verabreichung und eine Wiederholungsverabreichung der Zusammensetzung umfasst.

26. Immunogene Zusammensetzung zur Verwendung nach Anspruch 25, **dadurch gekennzeichnet, dass** die Immuntherapie gleichzeitig mit oder vor einer Anti-Krebs-Therapie durchgeführt wird.

## Claims

1. Immunogenic composition comprising a carrier and, as antigenic structure, conjugates comprising all or part of the amino acid sequences of at least one peptide derived from an extracellular loop of the P-170 protein, each peptide being combined with at least two molecules of fatty acid containing a carbon chain of between C12 and C24, **characterized in that** said peptide(s) is (are) combined with the molecules of fatty acids by means of one or more molecules of polyethylene glycol (PEG), linked to the lysine (K) residues which are in the N- and C-terminal positions of the amino acid sequences of at least one peptide derived from an extracellular loop of the P-170 protein, provided that the amino acid sequences allow, under suitable administration conditions, the induction of anti-P-170 antibodies.

2. Immunogenic composition according to Claim 1, **characterized in that** it allows the induction of anti-P-170 antibodies for the reversion of multidrug resistance appearing in a patient suffering from a cancer.

3. Immunogenic composition according to Claims 1 and 2, **characterized in that** the conjugates comprise all or part of the amino acid sequences of at least two extracellular loops of the P-170 protein.

4. Immunogenic composition according to Claims 1 and 2, **characterized in that** the conjugates comprise all or part of the amino acid sequences of at least three extracellular loops of the P-170 protein.

5. Immunogenic composition according to Claims 1 to 4, in which the conjugates comprise all or part of the amino acid sequences selected from the peptides derived from extracellular loops 1, 4 and 6 of the human P-170 protein, said conjugates allowing the induction of human anti-P-170 (anti-hpp) antibodies.

6. Immunogenic composition according to Claim 5, in which the conjugates comprise all or part of the amino acid sequences of the peptides derived from loop 1 of the human P-170 protein corresponding to the three peptides 1a, 1b and 1c, resulting from cleavage of said extracellular loop 1 at the glycosylation sites.

7. Immunogenic composition according to Claims 1 to 4, in which the conjugates comprise all or part of the amino acid sequences selected from the peptides derived from extracellular loops 1, 2 and 4 of the murine P-170 protein, said conjugates allowing the induction of murine anti-P-170 (anti-mpp) antibodies.

8. Immunogenic composition according to Claims 1 to 6, **characterized in that** all or part of the amino acid sequences of the peptides of the conjugates are, respectively, chosen from the following amino acid sequences:
for loop 1:
peptide 1 SEQ ID NO 4:
GEMTDIFANAGNLEDLLMSNITNRSDINDTGFFMNLEEDMTRYAYYYS
or peptide 1a SEQ ID NO 5: GEMTDIFANAGNLEDLLMS
or peptide 1b SEQ ID NO 6: NITNRSDINDTGFF
or peptide 1c SEQ ID NO 7: MNLEEDMTRYAYYYS
for loop 4: SEQ ID NO 8: FSRIIGVFTRIDDPETKRQNSNLFS
for loop 6: SEQ ID NO 10: FRFGAYLVAHKLMSFED
and/or a combination thereof, and/or the modified amino acid sequences, provided that the amino acid sequences thus modified allow, under suitable administration conditions, the induction of anti-P-170 antibodies.

9. Immunogenic composition according to Claims 1 to 8, **characterized in that** a molecule of PEG, of 1 to 8000 monomers, is used.

10. Immunogenic composition according to Claims 1 to 6, **characterized in that** all or part of the amino acid sequences of the peptides of the conjugates are, respectively, chosen from the following amino acid sequences:
for loop 1:
peptide 1 SEQ ID NO 1:
GNMTDSFTKAEASILPSITNQSGPNSTLIISNSSLEEE
for loop 2: SEQ ID NO 2: KVLTSFTNKELAYAK
for loop 4: SEQ ID NO 3: SRDDDMETKRQNEN
and/or a combination thereof, and/or the modified amino acid sequences, provided that the amino acid sequences thus modified allow, under suitable administration conditions, the induction of anti-P-170 antibodies.

11. Immunogenic composition according to Claims 1 to 10, **characterized in that** each peptide is covalently bonded to at least two of the polyethylene glycol chains.

12. Immunogenic composition according to Claim 11, **characterized in that** the two polyethylene glycol chains are each coupled to a molecule of phosphatidyl ethanolamine.

13. Immunogenic composition according to Claims 1 to 12, **characterized in that** the carrier chosen to present the conjugates is chosen from the group consisting of liposomes, bacterial membrane proteins, enterobacterial Omp proteins, nanoparticles, micelles, gold particles, microbeads and virosomes.

14. Immunogenic composition according to Claim 13, **characterized in that** the carrier chosen consists of liposomes.

15. Immunogenic composition according to Claim 14, in which the conjugates and the liposomes are in a molar ratio of between 1/10 and 1/1000, preferably of 1/250.

16. Immunogenic composition according to Claims 14 and 15, in which the liposomes are preferably obtained by mixing the phospholipids dimyristoylphosphatidylcholine (DPMC), dimyristoylphosphatidylglycerol (DPMG) and cholesterol.

17. Immunogenic composition according to Claim 16, in which the mixture of DPMC, DPMG and cholesterol is in the proportions 0.9:0.1:0.7.

18. Immunogenic composition according to any one of Claims 1 to 17, also comprising at least one adjuvant.

19. Immunogenic composition according to Claim 18, in which the adjuvant is chosen from the group consisting of alum, calcium phosphate, interleukin 1, monophosphoryl lipid A (MPLA) and/or microcapsules of proteins and of polysaccharides.

20. Immunogenic composition according to Claim 19, in which the adjuvant is preferably alum.

21. Immunogenic composition according to any one of Claims 1 to 20, for use in the treatment and/or prevention of multidrug resistance appearing in a patient suffering from a cancer.

22. Immunogenic composition for use according to Claim 21, in which the cancer affects the kidney, the liver, the colon, the intestine, the prostate, the breast, the bladder, the brain, the blood (leukemia) and/or the medullary tissues (myeloma).

23. Immunogenic composition for use according to Claim 21, **characterized in that** the cancer is a solid tumor expressing the MDR1 gene encoding the human P-170 protein.

24. Immunogenic composition according to any one of Claims 1 to 20, for use in the treatment of multidrug resistance appearing in a patient suffering from a cancer treated by means of anticancer medicaments.

25. Immunogenic composition for use according to any one of Claims 21 to 24, **characterized in that** said treatment comprises a first administration, and a booster administration of said composition.

26. Immunogenic composition for use according to Claim 25, **characterized in that** the immunization method is carried out concomitantly with or preceding an anticancer treatment.


### SEQUENCE LISTING

<110> University of Reims AC Immune S.A.
<120> VACCIN THERAPEUTIQUE CIBLE CONTRE LA P-GLYCOPROTEINE 170 POUR INHIBER LA RESISTANCE MULTIDROGUES DANS LE TRAITEMENT DES CANCERS
<130> ACI PCT 001
<150> FR 03 091 88 000
   <151> 2003-07-25
<160> 17
<170> PatentIn version 3.1
<210> 1
   <211> 43
   <212> PRT
   <213> Homo sapiens
<400> 1
   Lys Gly Gly Asn Met Thr Asp Ser Phe Thr Lys Ala Glu Ala Ser Ile
   1               5                   10                  15
   Leu Pro Ser Ile Thr Asn Gln Ser Gly Pro Asn Ser Thr Leu Ile Ile
               20                  25                  30
   Ser Asn Ser Ser Leu Glu Glu Glu Gly Lys Lys
           35                  40
<210> 2
   <211> 20
   <212> PRT
   <213> Homo sapiens
<400> 2
   Lys Gly Lys Val Leu Thr Ser Phe Thr Asn Lys Glu Leu Ala Tyr Ala
   1               5                   10                  15
   Lys Gly Lys Lys
               20
<210> 3
   <211> 19
   <212> PRT
   <213> Homo sapiens
<400> 3
   Lys Gly Ser Arg Asp Asp Asp Met Glu Thr Lys Arg Gln Asn Glu Asn
   1               5                   10                  15
   Gly Lys Lys
<210> 4
   <211> 48
   <212> PRT
   <213> Artificial Sequence
<220>
   <223> peptide
<400> 4
   Gly Glu Met Thr Asp Ile Phe Ala Asn Ala Gly Asn Leu Glu Asp Leu
   1 5 10 15
   Leu Met Ser Asn Ile Thr Asn Arg Ser Asp Ile Asn Asp Thr Gly Phe
   20 25 30
   Phe Met Asn Leu Glu Glu Asp Met Thr Arg Tyr Ala Tyr Tyr Tyr Ser
   35 40 45
<210> 5
   <211> 19
   <212> PRT
   <213> Artificial Sequence
<220>
   <223> peptide
<400> 5
   Gly Glu Met Thr Asp Ile Phe Ala Asn Ala Gly Asn Leu Glu Asp Leu
   1 5 10 15
   Leu Met Ser
<210> 6
   <211> 14
   <212> PRT
   <213> Artificial Sequence
<220>
   <223> peptide
<400> 6
   Asn Ile Thr Asn Arg Ser Asp Ile Asn Asp Thr Gly Phe Phe
   1 5 10
<210> 7
   <211> 15
   <212> PRT
   <213> Artificial Sequence
<220>
   <223> peptide
<400> 7
   Met Asn Leu Glu Glu Asp Met Thr Arg Tyr Ala Tyr Tyr Tyr Ser
   1 5 10 15
<210> 8
   <211> 25
   <212> PRT
   <213> Artificial Sequence
<220>
   <223> peptide
<400> 8
   Phe Ser Arg Ile Ile Gly Val Phe Thr Arg Ile Asp Asp Pro Glu Thr
   1 5 10 15
   Lys Arg Gln Asn Ser Asn Leu Phe Ser
   20 25
<210> 9
   <211> 18
   <212> PRT
   <213> Artificial Sequence
<220>
   <223> peptide
<400> 9
   Phe Thr Arg Ile Asp Asp Pro Glu Thr Lys Arg Gln Asn Ser Asn Leu
   1 5 10 15
   Phe Ser
<210> 10
   <211> 17
   <212> PRT
   <213> Artificial Sequence
<220>
   <223> peptide
<400> 10
   Phe Arg Phe Gly Ala Tyr Leu Val Ala His Lys Leu Met Ser Phe Glu
   1 5 10 15
   Asp
<210> 11
   <211> 53
   <212> PRT
   <213> Homo sapiens
<400> 11
   Lys Gly Gly Glu Met Thr Asp Ile Phe Ala Asn Ala Gly Asn Leu Glu
   1 5 10 15
   Asp Leu Leu Met Ser Asn Ile Thr Asn Arg Ser Asp Ile Asn Asp Thr
   20 25 30
   Gly Phe Phe Met Asn Leu Glu Glu Asp Met Thr Arg Tyr Ala Tyr Tyr
   35 40 45
   Tyr Ser Gly Lys Lys
   50
<210> 12
   <211> 24
   <212> PRT
   <213> Homo sapiens
<400> 12
   Lys Gly Gly Glu Met Thr Asp Ile Phe Ala Asn Ala Gly Asn Leu Glu
   1 5 10 15
   Asp Leu Leu Met Ser Gly Lys Lys
   20
<210> 13
   <211> 19
   <212> PRT
   <213> Homo sapiens
<400> 13
   Lys Gly Asn Ile Thr Asn Arg Ser Asp Ile Asn Asp Thr Gly Phe Phe
   1 5 10 15
   Gly Lys Lys
<210> 14
   <211> 20
   <212> PRT
   <213> Homo sapiens
<400> 14
   Lys Gly Met Asn Leu Glu Glu Asp Met Thr Arg Tyr Ala Tyr Tyr Tyr
   1 5 10 15
   Ser Gly Lys Lys
   20
<210> 15
   <211> 30
   <212> PRT
   <213> Homo sapiens
<400> 15
   Lys Gly Phe Ser Arg Ile Ile Gly Val Phe Thr Arg Ile Asp Asp Pro
   1 5 10 15
   Glu Thr Lys Arg Gln Asn Ser Asn Leu Phe Ser Gly Lys Lys
   20 25 30
<210> 16
   <211> 23
   <212> PRT
   <213> Homo sapiens
<400> 16
   Lys Gly Phe Thr Arg Ile Asp Asp Pro Glu Thr Lys Arg Gln Asn Ser
   1 5 10 15
   Asn Leu Phe Ser Gly Lys Lys
   20
<210> 17
   <211> 22
   <212> PRT
   <213> Homo sapiens
<400> 17
   Lys Gly Phe Arg Phe Gly Ala Tyr Leu Val Ala His Lys Leu Met Ser
   1 5 10 15
   Phe Glu Asp Gly Lys Lys
   20